# EUROPEAN PATENT APPLICATION

(11) **EP 4 509 107 A1**
(43) Date of publication of application: **19.02.2025**
(21) Application number: 23871516.3
(22) Date of filing: 10.08.2023
(51) Int. Cl.: A61F 13/53, A61F 13/535, A61F 13/536, A61F 13/539

(54) **ABSORBENT ARTICLE**

(30) Priority: 28.09.2022 JP 2022155164
(71) Applicant: Daio Paper Corporation, Ehime 799-0492 (JP)
(72) Inventor: YAMASHITA, Yuichi, Shikokuchuo-shi, Ehime 799-0113 (JP); TAKAGI, Yurika, Shikokuchuo-shi, Ehime 799-0113 (JP)
(74) Representative: Williams Powell
(86) International application number: PCT/JP2023/029264
(87) International publication number: WO 2024/070274

(57) **Abstract**

The problem is to improve shape maintainability of an absorber and prevent escape of super absorbent polymer particles. The above problem is solved by the following solution. An absorbent element 50, which includes an absorber 56 and a wrapping nonwoven fabric 58 wrapping the absorber 56, is provided. The absorber 56 has a layer formed by accumulating a mixture of pulp fibers and super absorbent polymer particles. The wrapping nonwoven fabric 58 includes a meltblown layer formed of fibers having an average fiber diameter of 5 um or less, and a stack of five sheets of the wrapping nonwoven fabrics 58 has an air permeability of 25.5 to 70.0 cm³/(cm²·s), measured according to Method A (Frazier method) specified in JIS L 1096 : 2010. The absorbent element 50 is provided with compressed portions 51 arranged at intervals and compressed in a thickness direction so as to be depressed from an underside surface of the absorbent element 50 into the absorber 56, and the absorbent element 50 is free of compressed portions 51 compressed in the thickness direction so as to be depressed from a top surface of the absorbent element 50 into the absorber 56.

## Description

### Technical Field

The present invention relates to an absorbent article such as a disposable diaper.

### Background Art

In an absorbent article, it is common to use an absorber formed by accumulating a mixture of pulp fibers and super absorbent polymer particles. Further, in general, such an absorber is incorporated in the disposable diaper in the form of an absorbent element in which the absorber is wound by a wrapping sheet made of crepe tissue, etc. to improve shape maintainability of the absorber during manufacture and after manufacture (see Patent Literature 1, 2, for example).

An absorbent element of an absorbent article receives forces in various directions from both sides in a width direction due to movement of wearer's legs such as walking while being interposed between both the legs. Thus, in a crotch part, an excellent fitting property of the absorbent element is required, and further, in order to suppress shape deformation such as twisting and cracking of the absorber, shape maintainability is also required. Additionally, it is needless to say that in the crotch part of the disposable diaper, an absorption performance of the absorbent element should be secured.

As a method for improving shape maintainability of an absorber, it is known that compressed portions, which are formed by compressing an absorbent element in a thickness direction by means of embossing or the like, are provided in a lattice shaped pattern or the like (see Patent Literature 1, 2 for example). However, if a wrapping sheet wrapping the absorber is crepe tissue, when the compressed portions are formed, the crepe tissue may be broken at edges of the compressed portions or between the compressed portions adjacent to each other. In addition, if the crepe tissue absorbs excrement liquid, it becomes easily-broken in a wet state due to decreased strength thereof. When the wrapping sheet is thus broken, super absorbent polymer particles may escape through the broken part so as to be exposed on a top surface of the absorbent article. Therefore, this method is unfavorable.

It is known that a fine nonwoven fabric is also used as the wrapping sheet. Since a nonwoven fabric is hardly-broken even in a wet state, it has been expected that escape of the super absorbent polymer particles through the fine nonwoven fabric can be suppressed effectively. However, even in a case where the fine nonwoven fabric is used as the wrapping sheet, it is confirmed that an amount of the super absorbent polymer particles escaped through the wrapping sheet is actually larger than anticipated.

### Citation List

### Patent Literature

Patent Literature 1: JP 2021-000236 A
Patent Literature 1: JP 6380454 B

### Summary of Invention

### Technical Problem

Therefore, a main object of the present invention is to make it difficult for super absorbent polymer particles to be exposed on a top surface of an absorbent article, while shape maintainability of an absorber is improved.

### Solution to Problem

The present inventors have conducted intensive studies and found that in a case where a wrapping sheet is made of a nonwoven fabric, when compressed portions are formed, the nonwoven fabric is elongated at edges of the compressed portions or between the compressed portions adjacent to each other such that interstices between adjacent fibers of the nonwoven fabric are enlarged, and therefore super absorbent polymer particles escape easily through the enlarged interstices. An absorbent article described below is based on such finding.

### <First aspect>

An absorbent article including:
a crotch part;
a front side part which extends forward from the crotch part;
a back side part which extends backward from the crotch part; and
an absorbent element including an absorber and a wrapping nonwoven fabric wrapping the absorber, the absorbent element being provided in a range in a front-back direction including the crotch part,
wherein the absorber has a layer formed by accumulating a mixture of pulp fibers and super absorbent polymer particles,
the wrapping nonwoven fabric includes a meltblown layer formed of fibers having an average fiber diameter of 5 um or less,
the wrapping nonwoven fabrics has an air permeability of 25.5 to 70.0 cm³/(cm²·s), measured according to Method A (Frazier method) specified in JIS L 1096 : 2010, in a state in which five layers of the nonwoven fabrics,
the absorbent element is provided with compressed portions arranged at intervals and compressed in a thickness direction so as to be depressed from an underside surface of the absorbent element into the absorber; and
the absorbent element is free of compressed portions compressed in the thickness direction so as to be depressed from a top surface of the absorbent element into the absorber.

### (Effect)

The present absorbent article is characterized in that
(a) since the nonwoven fabric including the dense meltblown layer is used as a wrapping material wrapping the absorber, problems (for example, the wrapping material would be broken during manufacturing and during use) can be solved, although such problems would be caused by crepe tissue used as the wrapping material,
(b) since the absorbent element is provided with the compressed portions depressed from the underside surface of the absorbent element into the absorber, shape maintainability and the like of the absorber can be improved, and
(c) since the absorbent element is free of the compressed portions depressed from the top surface of the absorbent element into the absorber, in a part of the wrapping nonwoven fabric which covers the top surface of the absorbent element (namely, this part refers to a top surface-part of the wrapping nonwoven fabric), interstices between the adjacent fibers thereof are not enlarged. Thus, it becomes possible to keep a state where it is difficult for the super absorbent polymer particles to pass through the top surface-part of the wrapping nonwoven fabric as a wrapping sheet.

Therefore, in the present absorbent article, as before, the shape maintainability and the like of the absorber is improved by providing the compressed portions, and in addition to this, the escape of the super absorbent polymer particles can be suppressed effectively, although such escape has been a problem caused by the compressed portions formed in a conventional manner.

Noted that an air permeability provides an indication of easiness of the escape of the super absorbent polymer particles.

### <Second aspect>

The absorbent article according to the first aspect,
wherein the wrapping nonwoven fabric includes the one or plural meltblown layers and one or plural protective layers,
constituent fibers of the protective layer have an average fiber diameter of 10 to 25 um,
the wrapping nonwoven fabric has an elongation rate of 20 to 100% in the front-back direction, measured according to a normal time method specified in JIS L 1913 : 2010, and
the wrapping nonwoven fabric has an elongation rate of 20 to 110% in a width direction, measured according to the normal time method specified in JIS L 1913 : 2010.

### (Effect)

If the wrapping nonwoven fabric is not elongated easily, it becomes hard to form the reliable compressed portions (namely, it becomes hard to improve the shape maintainability), in addition to this, flexibility of the absorber is decreased. Accordingly, it is preferable that the wrapping nonwoven fabric is sufficiently thin as in this second aspect so as to be elongated easily. On the other hand, in such a sufficiently thin nonwoven fabric, the interstices between the adjacent fibers thereof would tend to be enlarged by forming the compressed portions. In this respect, characteristics of the present invention explained in the above items (a) to (c) make it possible to suppress the escape of the super absorbent polymer particles while the flexibility and shape maintainability of the absorber can be improved.

### <Third aspect>

The absorbent article according to the first or second aspect,
wherein, in the absorber, the pulp fibers have a basis weight of 100 to 450 g/m²,
in the absorber, a weight ratio of pulp fibers : super absorbent polymer particles is 30 : 70 to 70 : 30,
a maximum thickness of the absorbent element is 4 to 35 mm, and
a thickness of each of the compressed portions is 15 to 35% of the maximum thickness of the absorbent element.

### (Effect)

Material composition of the absorber and compression degree of the compressed portions can be determined as appropriate, but it is preferable that they are within ranges stated as in this third aspect.

### <Fourth aspect>

The absorbent article according to the third aspect,
wherein a width of each of the compressed portions is 1 to 3 mm, and
the compressed portions are formed in an oblique lattice shape including first portions each of which diagonally inclines at a clockwise angle of 40 to 50° with respect to the front-back direction in a plan view, and second portions each of which diagonally inclines at a counterclockwise angle of 40 to 50° with respect to the front-back direction in the plan view.

### (Effect)

Dimensions and shapes of the compressed portions can be determined as appropriate, but it is preferable that the compressed portions are formed in the oblique lattice shape as in this fourth aspect, because not only the shape maintainability and flexibility of the absorber, but also liquid diffusivity in the absorbent element is improved. Here, when the compressed portions are arranged so as to form a pattern stated in this fourth aspect, in particular, the wrapping nonwoven fabric tends to be elongated easily, thus the characteristics explained in the above items (a) to (c) become increasingly important.

### <Fifth aspect>

The absorbent article according to any one of the first to fourth aspects,
wherein the absorber includes, at a top surface thereof, a covering layer formed by accumulating only pulp fibers.

### (Effect)

It is preferable that the absorber includes the covering layer as in this fifth aspect, because the covering layer makes it difficult for the super absorbent polymer particles to escape through the top surface of the absorber.

### <sixth aspect>

The absorbent article according to any one of the first to fifth aspects,
wherein the wrapping nonwoven fabric has:
   a top surface-portion located on a top side of the absorber;
   a first underside-portion, which continues from the top surface-portion and goes around the absorber on a one side edge thereof to be folded back and extended inwardly on an underside thereof; and
   a second underside-portion, which continues from the top surface-portion and goes around the absorber on an other side edge thereof to be folded back and extended inwardly on the underside thereof,
a tip side portion of the first underside-portion and a tip side portion of the second underside-portion are overlapped with each other so as to form a stacked portion, and
all of the compressed portions are formed within the stacked portion.

### (Effect)

When the wrapping nonwoven fabric has the structure as in this sixth aspect, the escape of the super absorbent polymer particles through the underside surface of the absorbent element can be also suppressed.

### <Seventh aspect>

The absorbent article according to any one of the first to sixth aspects,
wherein an entire range in the front-back direction of a middle part of the absorbent element excluding both end portions thereof in the width direction is uncovered by any other member, such that the wrapping nonwoven fabric is exposed on a top surface of the absorbent article.

### (Effect)

In the present absorbent article, the escape of the super absorbent polymer particles through the wrapping nonwoven fabric can be suppressed as stated above. Therefore, as in this seventh aspect, it is possible to omit a member such as a top sheet, although the top sheet is provided for covering the top surface of the absorbent element in the conventional absorbent article. As a result, in the case of this seventh aspect, a significant cost reduction can be attained.

### <Eighth aspect>

The absorbent article according to any one of the first to seventh aspects,
wherein a liquid impervious sheet is attached to the underside surface of the absorbent element via a hot melt adhesive,
the liquid impervious sheet extends from a position on a front side of a front edge of the absorbent element to a position on a back side of a back edge of the absorbent element, and from a position on a left side of a left side edge of the absorbent element to a position on a right side of a right side edge of the absorbent element, and
the hot melt adhesive is applied from a position on a front side of a front edge of the absorber to a position on a back side of a back edge of the absorber, and from a position between a left side edge of the absorber and the left side edge of the absorbent element to a position between a right side edge of the absorber and the right side edge of the absorbent element.

### (Effect)

To attach the liquid impervious sheet to the underside surface of the absorbent element, if the hot melt adhesive is applied to a range as stated in this eighth aspect, decrease of flexibility of both the side edges of the absorbent element can be suppressed, and in addition to this, the escape of the super absorbent polymer particles through the underside surface of the absorbent element can be suppressed effectively.

### Advantageous Effects of Invention

The present invention provides advantages, for example, it is difficult for super absorbent polymer particles to expose on a top surface of an absorbent article, while shape maintainability of an absorber is improved.

### Brief Description of Drawings

Fig. 1 is a plan view illustrating an internal surface of an underpants-type disposable diaper in a spread state.
Fig. 2 is a plan view illustrating an external surface of the underpants-type disposable diaper in the spread state.
Fig. 3 is a cross-sectional view taken along 2-2 line of Fig. 1.
Fig. 4 is a cross-sectional view taken along 3-3 line of Fig. 1.
Fig. 5(a) is a cross-sectional view taken along 4-4 line of Fig. 1, and Fig. 5(b) is a cross-sectional view taken along 5-5 line of Fig. 1.
Fig. 6 is a perspective view of the underpants-type disposable diaper.
Fig. 7 is a plan view illustrating an external surface of an inner member together with an outline of an outer member in the spread state.
Fig. 8 is a plan view illustrating a top surface of an absorber together with an outline of a wrapping nonwoven fabric.
Fig. 9 is a cross-sectional view of an absorbent element.
Fig. 10 is a plan view of the absorbent element.
Fig. 11 is an enlarged view of a main part of a top surface of the absorbent element.
Fig. 12 is a plan view illustrating other examples of the absorber.
Fig. 13 is a cross-sectional view illustrating the absorbent element in a state where it is before the absorber is wrapped with the wrapping nonwoven fabric.
Fig. 14 is a cross-sectional view illustrating the absorbent element in a state where it is after the absorber is wrapped with the wrapping nonwoven fabric and before compressed portions are formed.
Fig. 15 is a cross-sectional view of another absorbent element.
Fig. 16 is a plan view of other absorbent elements.
Fig. 17 is a cross-sectional view of another example corresponding to the cross-sectional view taken along 2-2 line of Fig. 1.
Fig. 18 is a cross-sectional view of the other example corresponding to the cross-section view taken along 3-3 line of Fig. 1.
Fig. 19 is a cross-sectional view of another absorbent element.
Fig. 20 is a cross-sectional view of another absorbent element.
Fig. 21 is a schematic view illustrating a method for producing an absorbent element.
Fig. 22 is a schematic view of a test facility.
Fig. 23 is a drawing illustrating only a main part for explaining a pattern of friction.

### Description of Embodiments

Hereinafter, as an example of disposable diaper, an underpants-type disposable diaper will be described with reference to the accompanying drawings. Note that respective constituent members adjacent in a thickness direction are fixed or bonded to each other as necessary in a similar manner to a known diaper also at a portion other than a fixed or bonded portion described below. Each of dotted pattern regions in the cross-sectional views indicates an adhesive such as a hot melt adhesive as a fixing or bonding means. The hot melt adhesive can be applied by a known method such as slot application, bead application into a continuous line or dot line, spray application into a spiral shape, Z shape, wave shape, etc., pattern coating (transfer of a hot melt adhesive by a letterpress method) or the like. Alternatively, a fixed portion for an elastic member is formed, instead of or in addition to the above applications of the hot melt adhesive, by application of the hot melt adhesive to an outer peripheral surface of the elastic member, and the elastic member can be fixed to a member located adjacent thereto. Examples of the hot melt adhesive include an EVA-based agent, pressure sensitive adhesive rubber-based agent (elastomer-based agent), polyolefin-based agent, and polyester/polyamide-based agent, and these can be used without particular limitation. As the fixing or bonding means for bonding the respective constituent members to each other, a means by material welding such as heat sealing or ultrasonic sealing also can be used. In a portion where a liquid pervious property in the thickness direction is required, the respective constituent members adjacent in the thickness direction are fixed or bonded to each other in an intermittent pattern. For example, when such intermittent fixing or bonding is performed with the hot melt adhesive, intermittent pattern application in a spiral shape, Z shape, wave shape, or the like can be suitably used, and when application is performed in a range larger than an application width by one nozzle, intermittent pattern application in a spiral shape, Z shape, wave shape, or the like can be performed with or without a space in a width direction. As the bonding means for bonding the respective constituent members to each other, a means by material welding such as heat sealing or ultrasonic sealing also can be used.

In addition, as a nonwoven fabric in the following description, a known nonwoven fabric can be appropriately used according to a site or a purpose. Examples of a constituent fiber of the nonwoven fabric include a synthetic fiber such as a polyolefin-based fiber including polyethylene and polypropylene, a polyester-based fiber, or a polyamide-based fiber (including a composite fiber such as core-sheath in addition to a single component fiber), a regenerated fiber such as rayon or cupra, and a natural fiber such as cotton, and any of these fibers can be selected without particular limitation. It is also possible that these fibers are mixed and used. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), a hydrophobic fiber, or a water-repellent fiber (including a fiber that has become water-repellent by a water repellent agent). In addition, the nonwoven fabric is generally classified into a short fiber nonwoven fabric, a long fiber nonwoven fabric, a spunbonded nonwoven fabric, a meltblown nonwoven fabric, a spunlace nonwoven fabric, a thermal bond (air-through) nonwoven fabric, a needle punch nonwoven fabric, a point bond nonwoven fabric, a laminated nonwoven fabric (including an SMS nonwoven fabric, an SMMS nonwoven fabric, or the like in each of which a meltblown layer is interposed between spunbonded layers), and the like depending on a fiber length, a sheet forming method, a fiber bonding method, and a stacked structure, and any of these nonwoven fabrics can be used.

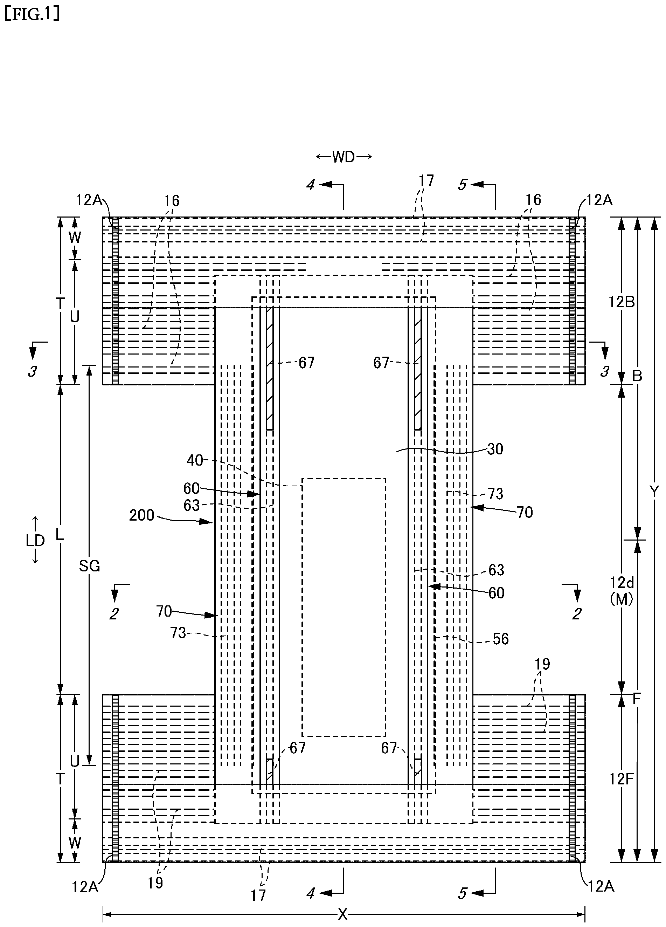

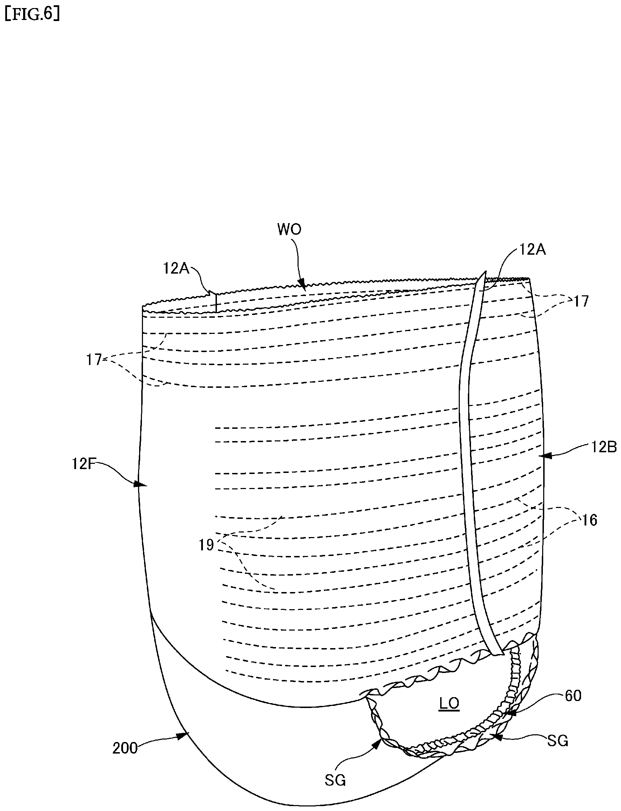

Fig. 1 to Fig. 6 illustrate an example of an underpants-type disposable diaper. The underpants-type disposable diaper includes: a rectangular shaped front outer member 12F forming a lower torso portion of a front body; a rectangular shaped back outer member 12B forming a lower torso portion of a back body; and an inner member 200 provided on an inner side of an outer member 12F, 12B to extend from the front outer member 12F to the back outer member 12B through a crotch part M. Both side portions of the front outer member 12F and both side portions of the back outer member 12B are bonded to each other to form side seal portions 12A, thereby forming a waist opening WO, which is formed by a front edge and a back edge of the outer member 12F, 12B and through which a wearer's lower torso passes, and leg openings LO, which are provided on both sides in the width direction WD of the inner member 200 so as to be surrounded by lower edges of the outer member 12F, 12B and side edges of the inner member 200, and through which wearer's legs pass, respectively. The inner member 200 is a portion absorbing and holding excrement e.g. urine and the like, and the outer member 12F, 12B is a portion for supporting the inner member 200 with respect to a wearer's body. A reference sign Y represents a maximum length of the diaper in a spread state (length in a front-back direction from an edge of the waist opening WO of the front body F to an edge of the waist opening WO of the back body B), and a reference sign X represents the maximum width of the diaper in the spread state.

The underpants-type disposable diaper includes a lower torso region T formed as a range in the front-back direction having the side seal portions 12A (a range in the front-back direction from the waist opening WO to upper ends of the leg openings LO) and an intermediate region L formed as a range in the front-back direction of a portion forming the leg openings LO (between a region in the front-back direction having the side seal portions 12A of the front body F and a region in the front-back direction having the side seal portions 12A of the back body B). A portion located as the lower torso region T of the front outer member 12F and the lower torso region T of the back outer member 12B, namely, as the lower torso portion of the front body and the lower torso portion of the back body may be conceptually divided into a "waist end portion" W forming an edge portion of the waist opening and an "under-waist end portion" U corresponding to a portion below the waist end portion. Normally, in a case where the portion located as the lower torso portion of the front body and the lower torso portion of the back body has a boundary in which a stretching force in the width direction WD changes (for example, the fineness of elastic members or the stretch rate thereof changes), a portion closer to the waist opening WO than the boundary closest to the waist opening WO is set as the waist end portion W. In a case where there is no such a boundary, a waist end side extending portion 12E, which extends toward the waist opening WO than an absorber 56 or the inner member 200, is set as the waist end portion W. The lengths in the front-back direction of the above portions vary depending on the size of a product and can be appropriately determined, and for example, the length of the waist end portion W can be 15 to 40 mm, and the length of the under-waist end portion U can be 65 to 120 mm. Meanwhile, the intermediate region L is narrowed on both side edges in substantially U shapes or curved shapes so as to follow peripheries of the wearer's legs, and both the side edges correspond to sites through which the wearer's legs are inserted. Therefore, the underpants-type disposable diaper in the spread state has a substantially hourglass shape as a whole.

### (Inner member)

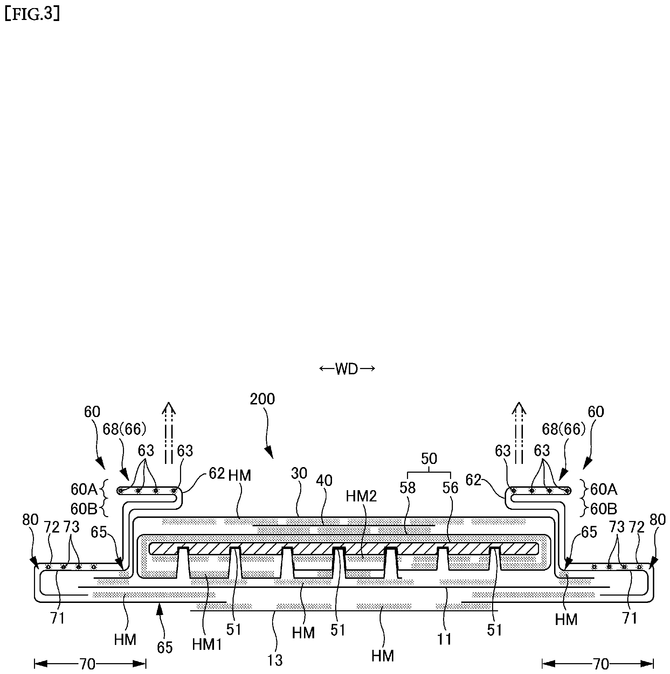

An arbitrary shape can be adopted for the inner member 200, and a rectangular shape is adopted in an illustrated example. As illustrated in Figs. 3 to 5, the inner member 200 includes a top sheet 30 corresponding to a wearer's body side, a liquid impervious sheet 11, and an absorbent element 50 interposed therebetween, and is a main unit member for performing an absorption function. A reference sign 40 represents an intermediate sheet (second sheet) disposed between the top sheet 30 and the absorbent element 50 in order to rapidly transfer a liquid that has passed through the top sheet 30 to the absorbent element 50. Reference signs 60 on both sides represent rising gathers, which extend from both side portions of the inner member 200 so as to come into contact with the peripheries of the wearer's legs in order to prevent leakage of excrement into both sides of the inner member 200.

### (Top sheet)

The top sheet 30 transmits a liquid, and examples thereof include a perforated or imperforated nonwoven fabric, or a perforated plastic sheet. In addition, the top sheet 30 may be composed of a single sheet or a stacked sheet obtained by adhering two or more sheets to each other. Similarly, the top sheet 30 may be composed of a single sheet or two or more sheets in a plane direction.

Both side portions of the top sheet 30 may be folded back to an underside of the absorbent element 50 at side edges thereof, or may extend laterally beyond side edges of the absorbent element 50 without being folded back.

It is desirable that the top sheet 30 is fixed to members adjacent to the top sheet 30 on underside thereof by the hot melt adhesive or the bonding means by material welding such as heat sealing or ultrasonic sealing, for the purpose of preventing positional deviation of the top sheet 30 with respect to the members on the underside of the top sheet 30. In the illustrated example, the top sheet 30 is fixed to a top surface of the intermediate sheet 40 and a top surface of the wrapping nonwoven fabric 58 at portions thereof located on a top side of the absorber 56, by the hot melt adhesive applied to an underside surface of the top sheet 30.

### (Intermediate sheet)

In order to quickly transfer the liquid that has passed through the top sheet 30 to the absorber 56, it is possible to dispose the intermediate sheet (also referred to as "second sheet") 40 having a higher liquid transmission rate than the top sheet 30. The intermediate sheet 40 is used in order to rapidly transfer a liquid to the absorber to enhance absorption performance of the absorber 56, and to prevent a "returning" phenomenon of the absorbed liquid from the absorber. The intermediate sheet 40 can be omitted. In this case, the top sheet 30 can be also omitted.

Examples of the intermediate sheet 40 may be formed of a similar material to that of the top sheet 30, a spun lace nonwoven fabric, a spunbond nonwoven fabric, an SMS nonwoven fabric, a pulp nonwoven fabric, a mixed sheet of pulp and rayon, a point bond nonwoven fabric or crepe tissue. In particular, an air-through nonwoven fabric is bulky, and thus is preferable. It is preferable to use a composite fiber having a core-sheath structure for the air-through nonwoven fabric. In this case, a resin used for a core may be polypropylene (PP). However, polyester (PET) having high rigidity is preferable. A basis weight is preferably 17 to 80 g/m², more preferably 25 to 60 g/m². A fineness of a raw material fiber of the nonwoven fabric is preferably 2.0 to 10 dtex. In order to make the nonwoven fabric bulky, as mixed fibers of all or a part of raw material fibers, eccentric fibers having no core in the center, hollow fibers, eccentric and hollow fibers are also preferably used.

The intermediate sheet 40 in the illustrated example is disposed at the center so as to be shorter than a width of the absorber 56, but may be disposed over a maximum width of the absorber 56. A length in the front-back direction of the intermediate sheet 40 may be the same as the maximum length of the diaper, may be the same as a maximum length of the absorbent element 50, or may be within a range having a short length centered on a region in which a liquid is received.

It is desirable that the intermediate sheet 40 is fixed to members adjacent to the intermediate sheet 40 on underside thereof by the hot melt adhesive or the bonding means by material welding such as heat sealing or ultrasonic sealing, for the purpose of preventing positional deviation of the intermediate sheet 40 with respect to the members on the underside of the intermediate sheet 40. In the illustrated example, the intermediate sheet 40 is fixed to the top surface of the wrapping nonwoven fabric 58 at portions thereof located on the top side of the absorber 56, by the hot melt adhesive applied to an underside surface of the intermediate sheet 40.

### (Liquid impervious sheet)

A material of the liquid impervious sheet 11 is not particularly limited, but examples thereof include a plastic film made of a polyolefin-based resin such as polyethylene or polypropylene, a laminated nonwoven fabric having a plastic film disposed on a surface of a nonwoven fabric, and a stacked sheet obtained by superposing and bonding a nonwoven fabric or the like to a plastic film. A material having liquid impermeability and moisture permeability, which has been favorably used from the viewpoint of prevention of stuffiness, is preferably used for the liquid impervious sheet 11. As a moisture pervious plastic film, a microporous plastic film is widely used which is obtained by kneading an inorganic filler in a polyolefin-based resin such as polyethylene or polypropylene, molding the kneaded mixture into a sheet, and then stretching the sheet in one or two axial directions. In addition, a nonwoven fabric using a micro denier fiber, a nonwoven fabric that has reinforced leakproofness by reducing a space between fibers by heating or applying pressure, and a sheet that has become liquid impervious without using a plastic film by a method for applying a super absorbent polymer, a hydrophobic resin, or a water repellent agent can be used as the liquid impervious sheet 11. However, it is desirable to use a resin film in order to obtain sufficient bonding strength at the time of attaching to a cover nonwoven fabric 13 described later via the hot melt adhesive.

The liquid impervious sheet 11 may have a width housed in the underside of the absorbent element 50 as illustrated in the drawing, or may go around both sides of the absorbent element 50 and extend to both side portions on a surface of the absorbent element 50 on a top sheet 30 side in order to enhance leakproofness. Thus, an extending portion formed in this way has appropriately a width of about 5 to 20 mm on each of the left and the right.

### (Absorbent element)

The absorbent element 50 includes the absorber 56 and the wrapping nonwoven fabric 58 wrapping a whole of the absorber 56.

### (Absorber)

The absorber 56 is formed by accumulating a mixture of pulp fibers and super absorbent polymer particles. A basis weight of the pulp fibers may be set to, for example, about 100 to 450 g/m².

The super absorbent polymer particles include "powder" in addition to "particles". As the super absorbent polymer particles, those used for this type of disposable diaper may be used as they are. For example, it is desirable that the proportion of particles remaining on the sieve is 30 wt% or less by sieving (shaking for 5 minutes) using a 500 um standard sieve (JIS Z 8801-1: 2006), and it is desirable that the proportion of particles remaining on the sieve is 60 wt% or more by sieving (shaking for 5 minutes) using a 180 um standard sieve (JIS Z 8801-1: 2006).

As the super absorbent polymer particles, any material can be used without particular limitation, but those having a water absorption capacity of 40 g/g or more are suitable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shape of the super absorbent polymer particles, a usually used particulate material shape is suitable, but other shapes may also be used.

As the super absorbent polymer particles, for example, those having a water absorption speed of 70 seconds or less, particularly 40 seconds or less are suitably used. When the water absorption speed is too low, so-called returning, in which a liquid that has been supplied into the absorber 56 returns out of the absorber 56, tends to occur.

In addition, as the super absorbent polymer particles, those having a gel strength of 1000 Pa or more are preferably used. This makes it possible to effectively suppress a sticky feeling after liquid absorption, even in a case where the absorber 56 is made bulky.

A basis weight amount of the super absorbent polymer particles may be appropriately determined according to an absorption amount required for the use of the absorber 56. Therefore, although it cannot be said unconditionally, the basis weight amount may be set to 50 to 350 g/m². The basis weight of the polymer particles of less than 50 g/m² makes it difficult to secure the absorption amount. The basis weight of more than 350 g/m² saturates an effect.

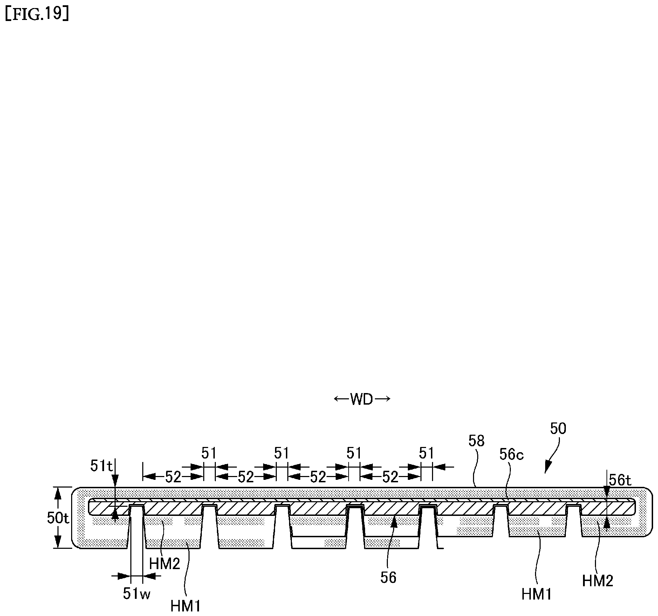

A ratio of fibers to super absorbent polymer particles in the absorber 56 is not particularly limited, and for example, a weight ratio of fibers : super absorbent polymer particles may be set to 40 : 60 to 65 : 35. In particular, it is preferable that the weight ratio of fibers : super absorbent polymer particles is set to 50 : 50 to 65 : 35, because in this ratio, shape maintainability of the absorber 56 is improved, absorption speed of the absorber 56 is increased, compressed portions 51 described later can be formed easily, the escape of the super absorbent polymer particles is unlikely to happen, and so on. In addition, it is usually preferable that the super absorbent polymer particles are almost uniformly distributed throughout the absorber 56. Meanwhile, from the viewpoint of suppression of the escape of the super absorbent polymer particles to the top side of the absorber, it is preferable that a content rate of the super absorbent polymer particles is reduced in a continuous or stepwise manner from an underside of the absorber 56 toward the top side thereof. In particular, as illustrated in Fig. 19, it is preferable that the absorber 56 includes, at a top surface thereof, a covering layer 56c formed by accumulating only pulp fibers. Also in a case where such covering layer 56c formed of only the pulp fibers is included, the weight ratio of fibers to super absorbent polymer particles preferably falls within the range specified above.

A thickness 56t of the absorber 56 is not particularly limited, but it may be set to 3 to 15 mm.

It is enough that the absorber 56 extends to both front and back sides of the crotch part M so as to cover it. In the underpants-type disposable diaper as in the present example, the absorber 56 preferably extends to a peripheral edge of the inner member 200 or the vicinity thereof in the front-back direction LD and the width direction WD. Note that a reference sign 56X represents the maximum width of the absorber 56.

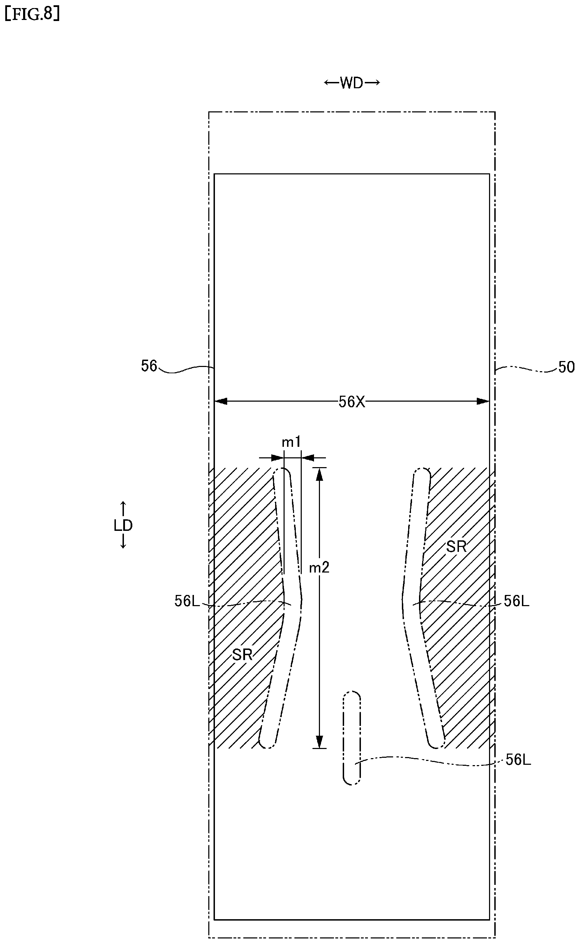

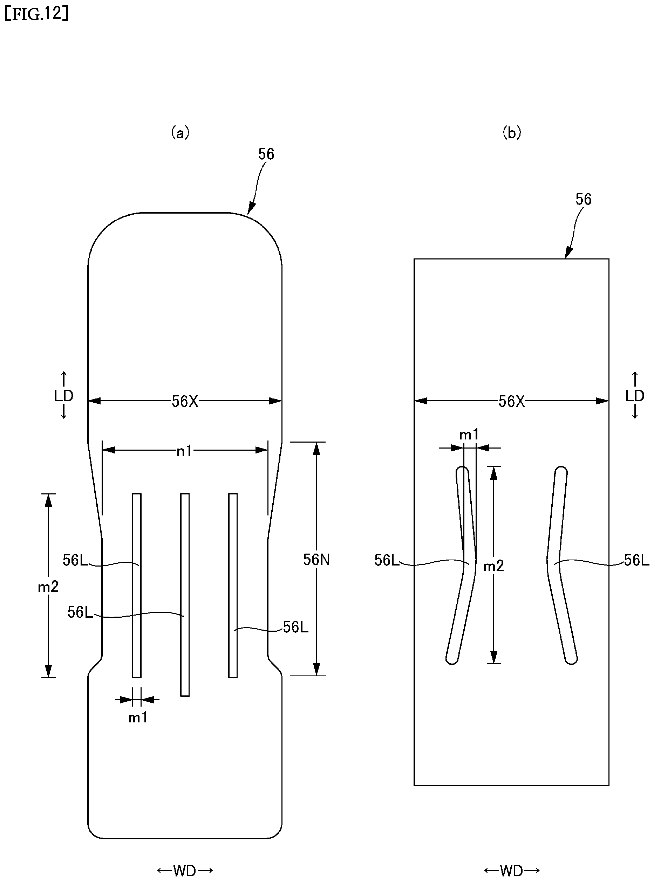

In order to make it easy to secure an absorption amount of the crotch part M, the absorber 56 may be preferably formed into a substantially rectangular shape as in the example illustrated in Fig. 8. In addition, as in the example illustrated in Fig. 12(a), in order to improve a fitting property of the crotch part M, it is possible that in the crotch part M, the absorber 56 is made to have a narrower portion by narrowing the absorber 56 so as to have a narrower width of the narrower portion than those of both front and back sides thereof. In this case, in order to make it easy to secure the absorption amount of the crotch part M, it is preferable that the absorber 56 has, in the crotch part M, a narrowest portion having a narrowest width n1 being equal to or more than 0.85 times the maximum width 56X of the absorber 56.

Incidentally, in a case where the absorber 56 has the narrower portion 56N, the crotch part M refers to a range in the front-back direction LD having the narrower portion 56N; in a case where the absorber 56 does not have the narrower portion 56N, and instead, as in the illustrated example, an outer shape of the diaper in the spread state has a narrower portion, the crotch part M refers to a range in the front-back direction LD having this narrower portion of the outer shape (in the illustrated example, the crotch part M is disposed between the front outer member 12F and the back outer member 12B); and in a case where the absorber 56 has neither of such narrowing portions, the crotch part M refers to a part disposed at a center in the front-back direction LD and a dimension in the front-back direction LD thereof is 20 to 30% of a maximum length of the product. A part extending forward from the crotch part M and a part extending backward therefrom refer to a front side part and a back side part respectively.

### (low-basis weight section)

In the crotch part M, the absorber 56 may have elongated low-basis weight sections 56L, which extend in the front-back direction LD on both sides thereof in the width direction WD or the like, as illustrated in Fig. 8 and Fig. 12. Alternatively, the low-basis weight section 56L can be omitted, as illustrated in Fig. 1 to Fig. 6. The low-basis weight section 56L refers to a section having a low basis weight and excludes a portion, which is just compressed in the thickness direction and whose basis weight is not changed as can be seen in a compressed portion 51 described later. As the low-basis weight section 56L, a slit, which penetrates the absorber in the thickness direction, can be adopted. However, as in the illustrated example, a recess in which a small amount of the mixture of the pulp fibers and the super absorbent polymer particles are accumulated is preferable as the low-basis weight section 56L because of easiness in securing the absorption amount. This recess may be formed on the top surface of the absorber 56 or on an underside surface thereof. Since the low-basis weight sections 56L are provided in the absorber 56, flexibility of the absorber 56 is enhanced along these low-basis weight sections 56L such that in the crotch part M, a fitting property of the absorbent element 50 can be improved. It is enough only that the total basis weight of the pulp fibers and the super absorbent polymer particles in the low-basis weight sections 56L is smaller than the total basis weight thereof in a portion of the absorber other than the low-basis weight sections 56L. For example, it is possible that the total basis weight thereof in the low-basis weight sections 56L is set to be 0.1 to 0.5 times the total basis weight thereof in the portion of the absorber other than the low-basis weight sections 56L.

As long as the low-basis weight section 56L extends in the front-back direction LD so as to be elongated, the low-basis weight section 56L may extend linearly along the front-back direction LD, or as in the illustrated example, the low-basis weight section 56L may be curved so that it is positioned laterally with increasing proximity to each end thereof along the front-back direction LD. Further, a front end portion and a back end portion of the low-basis weight section 56L can have appropriate shapes. For example, the low-basis weight section 56L has a linear shape as a whole as illustrated in Fig. 12(a). Alternatively, both the front end portion and the back end portion of the low-basis weight section 56L have convex shapes formed with curved lines (arcs of semi-circles, or the like) as in an example illustrated in Fig. 8, or the low-basis weight section 56L has, in each of the front end portion and the back end portion, two round corners and a linear shaped part therebetween, although not illustrated. A width m1 of the low-basis weight section 56L can be determined as appropriate, and for example, the width m1 can be 0.04 to 0.1 times the narrowest width n1 of the narrowest portion of the absorber 56 in the crotch part M (if the absorber 56 has a rectangular shape, the width n1 refers to the maximum width 56X). The width m1 of the low-basis weight section 56L can be constant or can be changed in a length direction thereof. A dimension in the front-back direction LD and an arrangement of the low-basis weight section 56L can be determined as appropriate. For example, a dimension m2 in the front-back direction LD of the low-basis weight section 56L can be 50 to 120%, preferably 50 to 80% of a dimension in the front-back direction LD of the crotch part M. Further, the low-basis weight section 56L may be housed in a range of the crotch part M, or may extend beyond the range of the crotch part M to the front side, the back side, or both the front and back sides thereof.

In the crotch part M, one low-basis weight section 56L may be provided on each of both (right and left) sides of the absorber 56 in the width direction WD and in addition to these low-basis weight sections 56L, one low-basis weight section 56L may be added so as to locate at a center of the absorber 56 in the width direction WD, as illustrated in Fig 8 and Fig. 12(a); only two low-basis weight sections 56L in total may be provided on both the sides of the absorber 56 in the width direction WD, as illustrated in Fig. 12(b); or only one low-basis weight section 56L may be provided so as to locate at the center in the width direction WD, although not illustrated.

### (Compressed portion)

The absorbent element 50, as illustrated in Fig. 3, Fig. 4 and Fig. 9, is provided with compressed portions 51 arranged at intervals and compressed in the thickness direction so as to be depressed from an underside surface of the absorbent element 50 into the absorber 56. On the other hand, the absorbent element 50 is free of compressed portions 51 compressed in the thickness direction so as to be depressed from a top surface of the absorbent element 50 into the absorber 56. The compressed portions 51 are high-density portions, which are formed by pressing (applying direct pressure to) the absorbent element 50 such that these high density portions have the substantially equal thickness to each other and locate at bottoms of depressed portions. Hereinafter, portions other than the compressed portions 51 are referred to as non-compressed portions 52. The non-compressed portion 52 has a larger thickness and a lower density than the compressed portion 51. However, even in the non-compressed portion 52, in the vicinity of periphery of the compressed portion 51, the absorber 56 and the wrapping nonwoven fabric are deformed by the influence of deformation of the compressed portion 51. As a result, the non-compressed portion 52 becomes dense with increasing proximity to the compressed portion 51. In addition, during, after, or before forming the compressed portion 51, the whole of the non-compressed portion 52 may be compressed in the thickness direction, as long as the non-compressed portion 52 has a larger thickness and a lower density than the compressed portion 51. Shapes and arrangements of the non-compressed portions 52 may be determined depending on those of the compressed portions 51, and the non-compressed portions 52 may be arranged in a staggered arrangement, a network arrangement, a lattice arrangement, a striped arrangement, or the like.

The absorbent element having such compressed portions 51 and non-compressed portions 52 may be produced by a known method such as embossing.

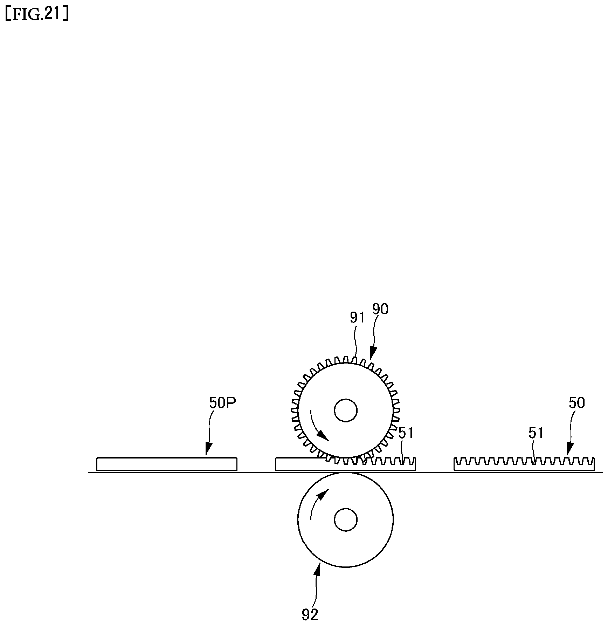

For example, the absorbent element 50 having the compressed portions 51 may be formed by the following three steps:
a first step of forming the absorber 56 by accumulating the mixture of the pulp fibers and the super absorbent polymer particles;
a second step of forming a wrapped body by wrapping the whole of the absorber 56 with the wrapping nonwoven fabric 58; and
a third step of passing the wrapped body 50P between an anvil roll 90, which has, on an outer peripheral surface thereof, a large number of projections 91 arranged at intervals in the same pattern as that of the compressed portions 51, and a smooth roll 92, which has a cylindrical outer peripheral surface (having no projections 91) facing the anvil roll 90, in a state where a surface of the wrapped body 50P to be the underside surface of the absorbent element 50 is on the anvil roll 90 side, and pressing the wrapped body 50P at portions thereof interposed between the large number of protrusions 91 of the anvil roll 90 and the smooth roll 92, respectively, such that the compressed portions 51 are formed, as illustrated in Fig. 21. In forming the compressed portions 51, either one or both of the anvil roll 90 and the smooth roll 92 may be heated, or both of these rolls 90, 92 may not be heated. In the third step, it is possible that the wrapped body 50P is pressed only at the portions thereof interposed between the large number of protrusions 91 and the smooth roll 92, alternatively, it is also possible that, the wrapped body 50P is entirely pressed, and at the same time, only the portions thereof interposed between the large number of protrusions 91 and the smooth roll 92 are more deeply pressed to the innermost.

A thickness 50t of the absorbent element 50 and a thickness 51t of the compressed portion 51 can be determined as appropriate. For example, a maximum value of the thickness 50t of the absorbent element 50 (a maximum value of a thickness of the non-compressed portion 52) may be set to 4 to 35 mm and in particular, it is preferably set to 5 to 13 mm. The thickness 51t of the compressed portion 51 can be determined as appropriate, but in a normal case, the thickness 51t is preferably 15 to 35% of the maximum value of the thickness 50t of the absorbent element 50.

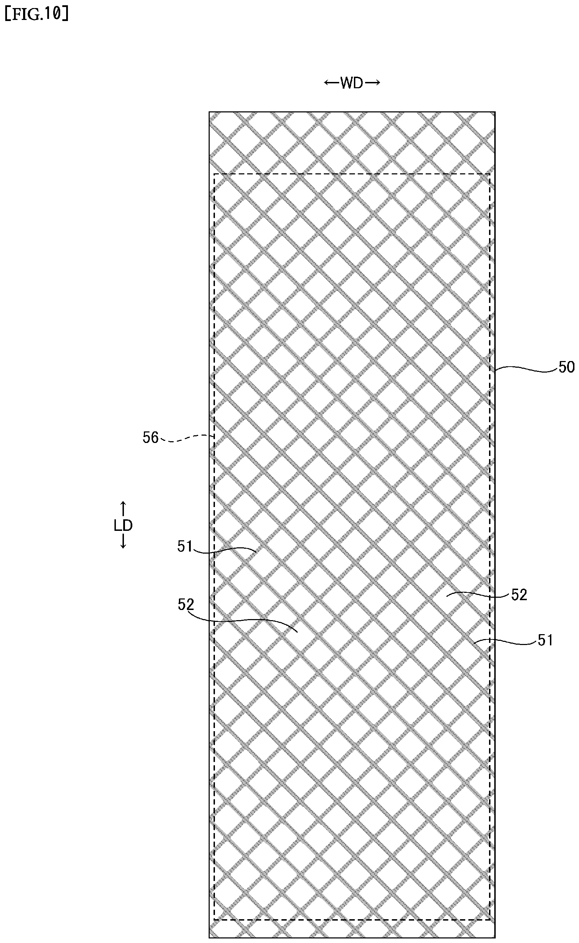

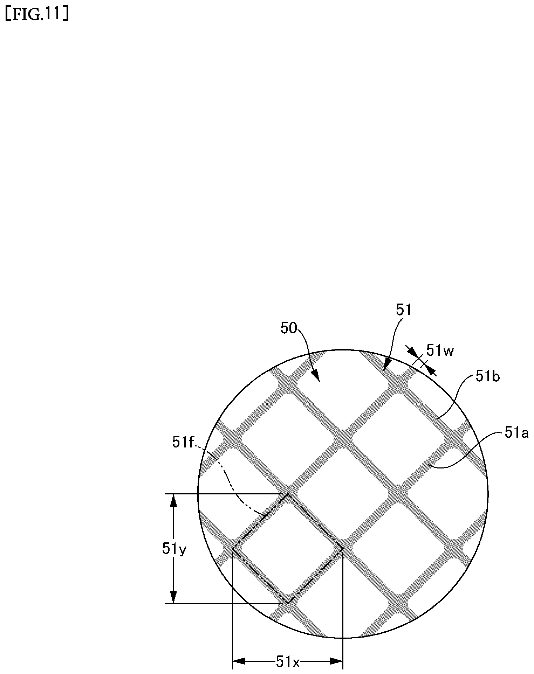

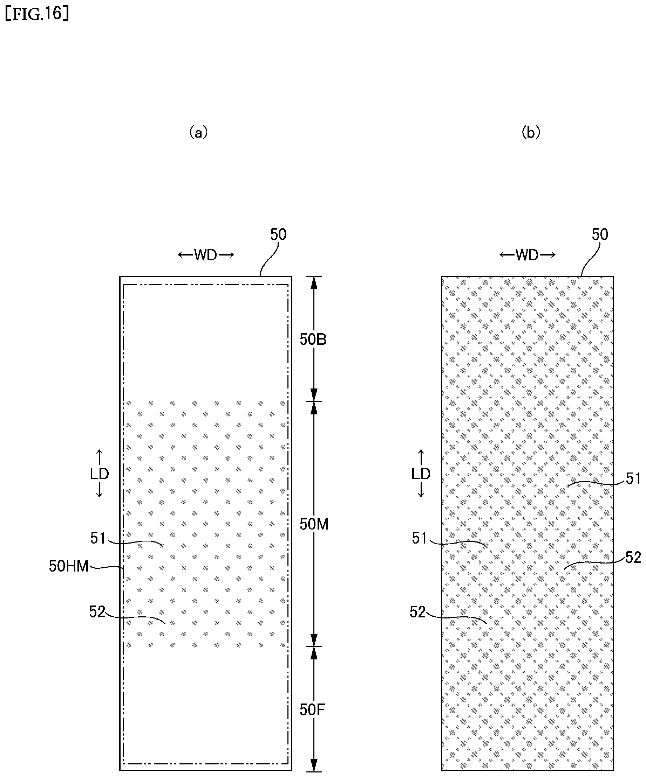

The compressed portions 51 may be arranged in a regular or irregular pattern as appropriate. Examples of patterns of the compressed portions 51 include a pattern in which the compressed portions 51 formed into continuous lines are arranged in a lattice shape as illustrated in Fig. 10 and Fig. 11; a pattern in which the compressed portions 51 formed into dotted lines are arranged in a lattice shape as illustrated in Fig. 16(b); a pattern in which the compressed portions 51 formed into continuous or dotted lines are arranged in a striped (vertical-striped, horizontal-striped, etc.) shape, although not illustrated; a pattern in which the compressed portions 51 formed into continuous or dotted lines are arranged in a matrix or staggered shape, as illustrated in Fig. 16(a), and the like.

In one preferable pattern of the compressed portions 51, as the example illustrated in Fig. 10 and Fig. 11, the compressed portions 51 include first portions 51a each of which extends diagonally in a direction inclining at a clockwise angle of 40 to 50° with respect to the front-back direction LD in a plan view, and second portions 51b each of which extends diagonally in a direction inclining at a counterclockwise angle of 40 to 50° with respect to the front-back direction LD in the plan view such that the compressed portions 51 have an oblique lattice shaped pattern. In this case, a shape of a unit frame 51f becomes a substantial rhombus shape. Dimensions of the unit frames 51f can be determined as appropriate and for example, a dimension 51x in the width direction WD of the unit frame 51f can be set to about 15 to 20 mm. In addition, a dimension 51y in the front-back direction LD of the unit frame 51f can be set to about 15 to 20 mm.

When the compressed portions 51 are formed into the continuous or dotted lines, a width 51w of each of the compressed portions 51 (a width of a bottom portion of a depressed portion formed in the absorbent element 50) can be determined appropriately, but in a normal case, it is preferably set to about 1 to 3 mm. In addition, when the compressed portions 51 are formed into dots, a maximum diameter (a maximum diameter of a bottom portion of the depressed portion formed in the absorbent element 50) can be determined appropriately, but in a normal case, it is preferably set to about 1 to 3 mm.

The area ratio of the compressed portions 51 (bottom portions of depressed portions) to the underside surface of the absorbent element 50 can be determined as appropriate. However, if the compressed portions 51 are arranged too densely, the absorbent element 50 becomes hard, thus this area ratio is preferably set to about 10 to 14%.

It is possible that the compressed portions 51 are provided over the whole underside surface of the absorbent element 50. Alternatively, it is also possible that the compressed portions 51 are provided only in a part of the underside surface thereof. For example, it is one of preferable examples that the compressed portions 51 are provided only in an intermediate part 50M in the front-back direction LD including the crotch part M, and are not provided both in a part 50F extending forward from the intermediate part 50M and a part 50B extending backward therefrom as illustrated in Fig. 16(a). The reason why this example is preferable is as follows. The intermediate part 50M of the absorbent element 50 receives forces in various directions from both sides in the width direction WD due to movement of the wearer's legs such as walking while being interposed between both the legs. In order to cope with this, the shape maintainability of the absorbent element 50 is desirably improved due to the compressed portions 51 provided in the intermediate part 50M. In addition to this, since the compressed portions 51 are not provided in both the other parts 50F and 50B than the intermediate part 50M, a protection effectiveness against the escape of the super absorbent polymer particles is desirably enhanced.

Further, it is preferable that the absorbent element 50 is, almost up to the both side edges thereof, securely fixed to a member located adjacent to the underside of the absorbent element 50 (the liquid impervious sheet 11 in the illustrated example). In this way, it becomes difficult for both side portions of the absorbent element 50 to deform, thus it becomes also difficult for the super absorbent polymer particles to escape at both these side portions. It is particularly preferable that, as in the example illustrated in Fig. 16(a), the compressed portions 51 are not provided in the vicinity (e.g., distance of about 5 to 10 mm in the width direction WD) of both side edges of the absorbent element 50, respectively, and both side edges of a bonding region 50HM, which is provided for fixing the absorbent element 50 and the member located adjacent to the underside of the absorbent element 50 (the liquid impervious sheet 11 in the illustrated example) to each other, are positioned between both the side edges of the absorbent element 50 and both the closest compressed portions 51 thereto, respectively.

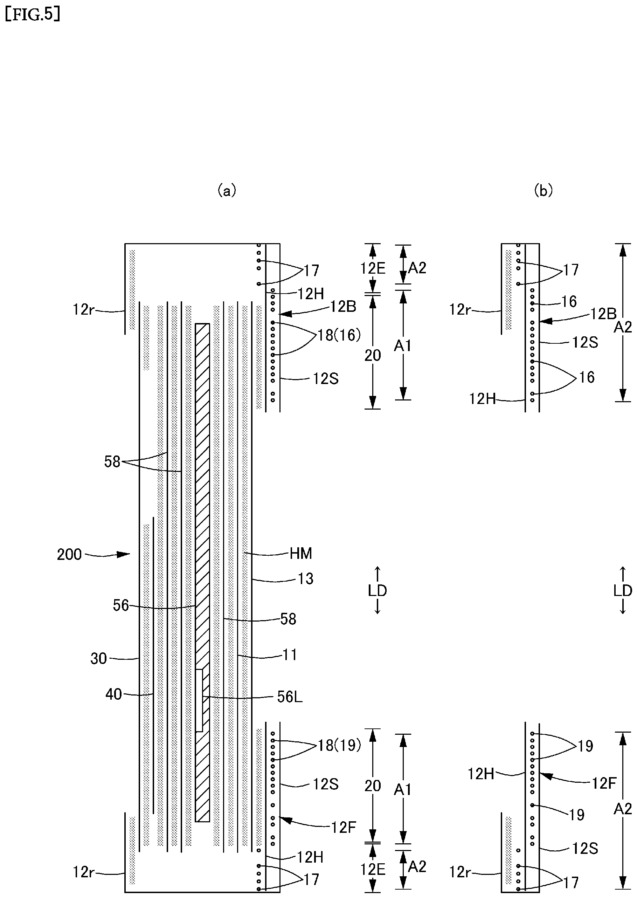

Moreover, as illustrated in Fig. 5, Fig. 17 and Fig. 18, in a case where the underside surface of the absorbent element 50 is attached to the liquid impervious sheet 11 via the hot melt adhesive HM, the liquid impervious sheet 11 extends from a position on a front side of a front edge of the absorbent element 50 to a position on a back side of a back edge of the absorbent element 50 and from a position on a left side of a left side edge of the absorbent element 50 to a position on a right side of a right side edge of the absorbent element 50, it is preferable that the hot melt adhesive HM is applied from a position on a front side of a front edge of the absorber 56 to a position on a back side of a back edge of the absorber 56 and from a position between a left side edge of the absorber 56 and the left side edge of the absorbent element 50 to a position between a right side edge of the absorber 56 and the right side edge of the absorbent element 50. In this way, decrease of flexibility of both the side edges of the absorbent element can be suppressed, and at the same time, the escape of the super absorbent polymer particles through the underside surface of the absorbent element can be suppressed effectively. As long as the hot melt adhesive HM is applied from the position on the front side of the front edge of the absorber 56 to the position on the back side of the back edge of the absorber 56, it is possible that the hot melt adhesive HM is applied from the position between the front edge of the absorber 56 and the front edge of the absorbent element 50 to the position between the back edge of the absorber 56 and the back edge of the absorbent element 50, alternatively it is also possible that the hot melt adhesive HM is applied to the position on the front side of the front edge of the absorbent element 50 to the back side of the back edge of the absorbent element 50.

### (Wrapping nonwoven fabric)

It is preferable that the wrapping nonwoven fabric 58 includes the meltblown layer formed of fibers having an average fiber diameter of 5 um or less and a stack of five sheets of the wrapping nonwoven fabrics has an air permeability of 25.5 to 70.0 cm³/(cm²·s), measured according to Method A (Frazier method) specified in JIS L 1096 : 2010. It is more preferable that the stack of five sheets of the wrapping nonwoven fabrics has an air permeability of 40.0 to 70.0 cm³/(cm²·s). An air permeability can be measured by, for example, Air Permeability Tester (Model AP-500KZ4) of DAIEI KAGAKU SEIKI MFG. CO., LTD. The meltblown layer is a nonwoven fabric layer formed by spinning a molten resin material in a high-temperature airstream of high-speed, and then accumulating thus formed ultrafine fibers to be bonded to each other, as is well known. The constituent fibers of the meltblown layer are thin with an average fiber diameter of 10 um or less, the constituent fibers of the meltblown layer have long fiber lengths of 30 um or more and, the constituent fibers of the meltblown layer have small interstices between the adjacent fibers. Further, the average fiber diameter of the constituent fibers of the meltblown layer is preferably 2.5 um or less. Moreover, the average fiber diameter of the constituent fibers of the meltblown layer can be 1 um or more and preferably 2 um or more.

The total basis weight of the meltblown layer (in a case where plurality of meltblown layers are included, the sum of basis weights of all meltblown layers) and the total thickness (in a case where plurality of meltblown layers are included, the sum of thicknesses of all meltblown layers) can be determined appropriately. For example, the total basis weight of the meltblown layer may be set to 0.3 to 6 g/m² and the total thickness of the meltblown layer may be set to 0.01 to 0.35 mm.

In this way, since the nonwoven fabric including the dense meltblown layer is used as a wrapping material, problems caused by crepe tissue used as the wrapping material (for example, the wrapping material would be broken during manufacturing and during use) can be solved . At the same time, as stated before, since the absorbent element 50 is provided with the compressed portions 51 depressed from the underside surface of the absorbent element 50 into the absorber 56, the shape maintainability and the like of the absorber 56 can be improved. In addition to this, since the absorbent element 50 is free of the compressed portions 51 depressed from the top surface of the absorbent element 50 into the absorber 56, even in a case where the wrapping nonwoven fabric 58 having a requisite minimum thickness is used, in a part of the wrapping nonwoven fabric 58 which covers the top surface of the absorbent element 50 (namely, this part refers to a top surface-part of the wrapping nonwoven fabric 58), the interstices between the adjacent fibers thereof are not necessary to enlarge. Thus, it becomes possible to keep a state where it is difficult for the super absorbent polymer particles contained in the absorber 56 to pass through the top surface-part of the wrapping nonwoven fabric 58. Therefore, as before, the shape maintainability and the like of the absorber 56 is improved by providing the compressed portions 51, and in addition to this, the escape of the super absorbent polymer particles can be suppressed effectively, although such escape has been a problem caused by the compressed portions formed in a conventional manner.

As long as the wrapping nonwoven fabric 58 has the meltblown layer, the wrapping nonwoven fabric may be a single layer nonwoven fabric having only the meltblown layer, but it is preferable that the wrapping nonwoven fabric 58 is a laminated nonwoven fabric having one or plural meltblown layers and one or plural protective layers. The laminated nonwoven fabric may be formed by stacking webs as respective layers in order, and subjecting the laminated structure to thermal bonding (point bonding, hot air bonding, etc.), whereby fibers are bonded to each other and the respective layers are bonded to each other. Alternatively, nonwoven fabric layers as respective layers are individually formed (by forming webs and bonding fibers to each other), and then these nonwoven fabric layers are stacked and bonded to each other thermally or with an adhesive so as to form the laminated nonwoven fabric.

As the protective layer, a spunbonded layer may be preferably used. The spunbonded layer is a nonwoven fabric layer formed by extruding (spinning) a molted resin material through a nozzle to form filaments and then accumulating the filaments to be bonded to each other. Then, the constituent fibers of the spunbonded layer have the average fiber diameter larger than the constituent fibers of the meltblown layer. In addition, the constituent fibers of the spunbonded layer have fiber lengths longer than the constituent fibers of the meltblown layer. More specifically, as the wrapping nonwoven fabric 58, an SMS nonwoven fabric, an SSMMS nonwoven fabric, or the like can be used favorably in each of which at least one meltblown layer is interposed between a pair of spunbonded layers on top side and underside. Materials of the constituent fibers of each layer is not particularly limited. For example, as the material of the constituent fiber of the meltblown layer, polypropylene fibers, polyethylene/polypropylene bicomponent fibers or the like can be used, and as the material of the constituent fiber of the spunbonded layer, polypropylene fibers, polyethylene/polypropylene bicomponent fibers or the like can be used.

Fiber diameters of the constituent fibers of the protective layer, the total basis weight of the protective layer (in a case where plurality of protective layers are included, the sum of basis weights of all protective layers), and the total thickness (in a case where plurality of protective layers are included, the sum of thicknesses of all protective layers) can be determined appropriately. For example, the constituent fibers of the protective layer preferably have average fiber diameter of 10 to 25 um (particularly 15 to 20 um). In addition, the total basis weight of the protective layer is preferably set to 5 to 17.5 g/m² (particularly 5 to 10 g/m²) and the total thickness of the protective layer is preferably set to, 0.01 to 0.35 mm (particularly 0.1 to 0.2 mm).

The basis weight and the thickness of the wrapping nonwoven fabric 58 can be determined appropriately. For example, the total basis weight of the wrapping nonwoven fabric 58 is preferably set to 10 to 15 g/m² (particularly 10 to 13 g/m²). In addition, the thickness of the wrapping nonwoven fabric 58 is preferably set to 0.2 to 0.8 mm.

If the wrapping nonwoven fabric 58 is not elongated easily, it becomes hard to form the reliable compressed portions 51 (namely, it becomes hard to improve the shape maintainability). In addition to this, flexibility of the absorber 56 is decreased. Accordingly, it is preferable that the wrapping nonwoven fabric 58 has an elongation rate of 20 to 100%, particularly 30 to 60% in the front-back direction LD, measured according to a normal time method specified in JIS L 1913 : 2010, and the wrapping nonwoven fabric has an elongation rate of 20 to 110%, particularly 50 to 70% in the width direction WD, measured according to the normal time method specified in JIS L 1913 : 2010. In such a nonwoven fabric, the interstices between adjacent fibers thereof would tend to be enlarged easily by forming the compressed portions 51. However, in the present absorber, due to the above-mentioned characteristics related to the compressed portions 51 and the like, improvement in flexibility and shape maintainability of the absorber 56 can be obtained, and at the same time, the escape of the super absorbent polymer particles can be suppressed.

A wrapping mode with the wrapping nonwoven fabric 58 may be determined appropriately, and in view of ease of manufacturing, in view of limiting escape of the super absorbent polymer particles from front and back end edges of the wrapping nonwoven fabric 58 and the like, the following mode is preferable. Precisely, as in the illustrated example, the wrapping nonwoven fabric 58 winds around the absorber 56 in a tubular shape so as to surround the top surface, the underside surface, and both side surfaces thereof, while a front end edge portion and a back end edge portion of the wrapping nonwoven fabric 58 are made to protrude forward and backward from the absorber 56 respectively, and overwrapped portions of the wrapping nonwoven fabric 58 at the seam and the overwrapped portions of the wrapping nonwoven fabric 58 protruded forward and backward from the absorber 56, respectively, are bonded to each other by the hot melt adhesive or the bonding means such as material welding.

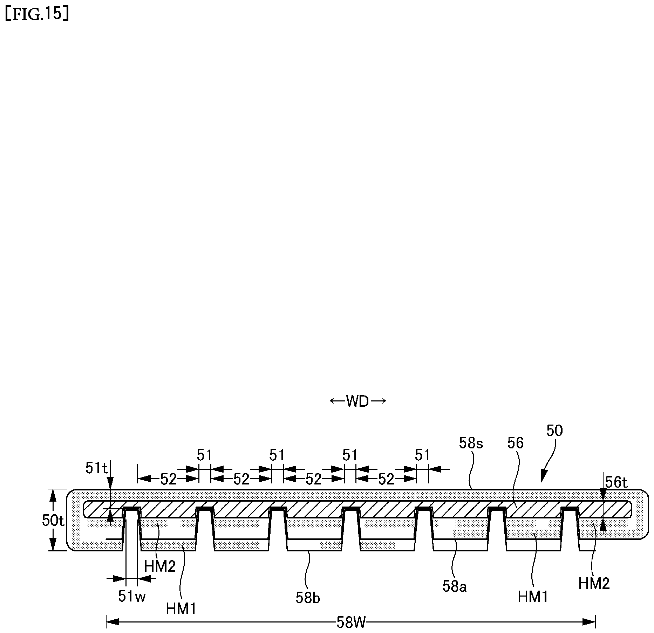

In particular, it is preferable that as illustrated in Fig. 15, the wrapping nonwoven fabric 58 has: a top surface-portion 58s located on the top side of the absorber 56; a first underside-portion 58a, which continues from the top surface-portion 58s and goes around the absorber 56 on one side edge thereof to be folded back and extended inwardly on an underside thereof; and a second underside-portion 58b, which continues from the top surface-portion 58s and goes around the absorber 56 on the other side edge thereof to be folded back and extended inwardly on the underside thereof, a tip side portion of the first underside-portion 58a and a tip side portion of the second underside-portion 58b are overlapped with each other so as to form a stacked portion 58W, and all of the compressed portions 51 are formed within the stacked portion 58W, because the escape of the super absorbent polymer particles through the underside surface of the absorbent element 50 can be also suppressed.

The shape maintainability of the absorbent element 50 due to the compressed portions 51 depends on maintainability of the compressed portions 51 themselves. Further, this maintainability of the compressed portions 51 themselves depends on the shape maintainability of the absorber 56 itself and bonding strength between the wrapping nonwoven fabric 58 and the absorber 56 in the compressed portions 51. Then, in a case where the wrapping nonwoven fabric 58 and the absorber 56 are bonded to each other by hot melt adhesives HM1 and HM2, since a surface area of the absorber is increased due to the compressed portions 51 formed thereon, a larger amount of the hot melt adhesives HM1 and HM2 than a normal amount is required in order to secure sufficiently the bonding strength between the wrapping nonwoven fabric 58 and the absorber 56 and the shape maintainability of the absorber 56 itself. Therefore, it is preferable that across at least an entire region having the compressed portions, an internal surface of the wrapping nonwoven fabric 58 and the underside surface of the absorber 56 are bonded to each other by 5.0 to 20.0 g/m², particularly 7.5 to 15.0 g/m² of the hot melt adhesives HM1, HM2.

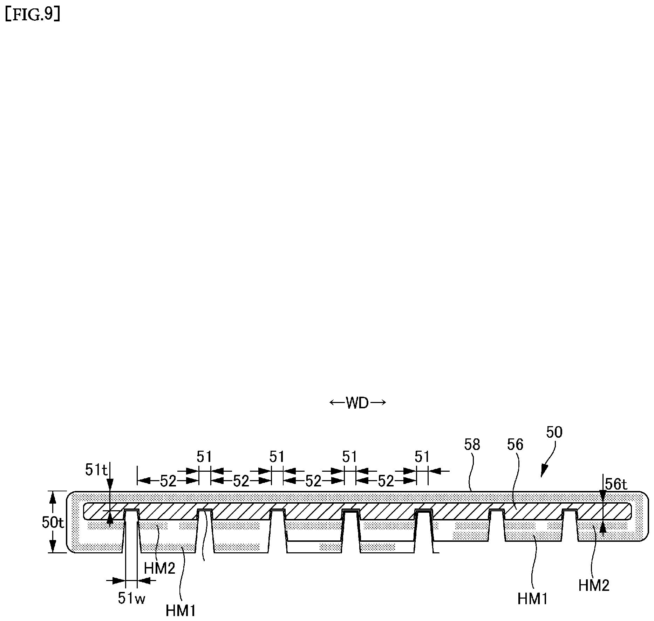

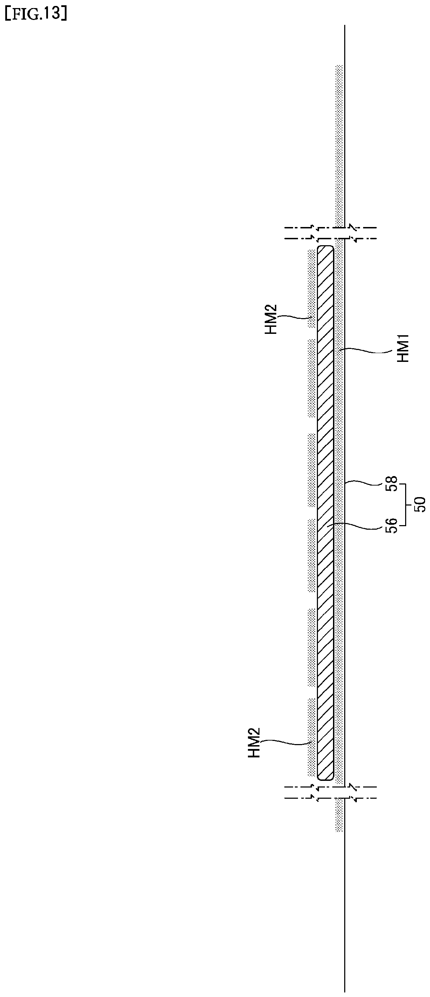

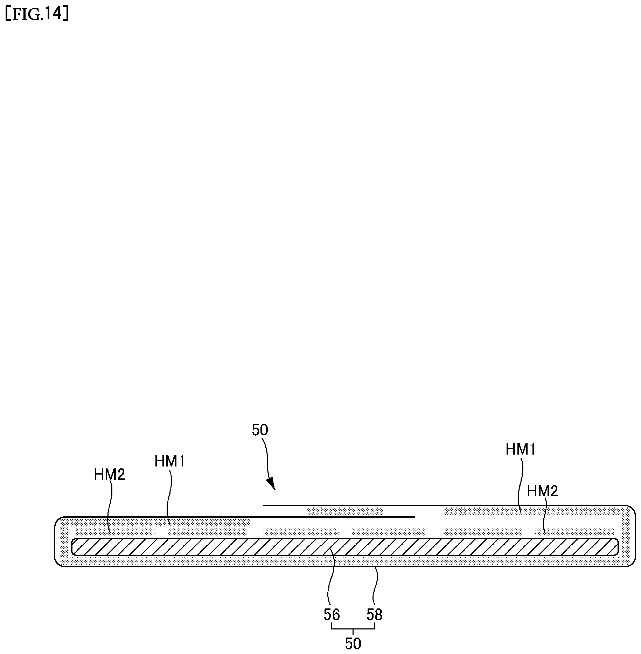

For example, as illustrated in Fig. 9, in a case where the depressed portions are formed by the compressed portions 51 on the underside surface of the absorber 56, one layer of the hot melt adhesive HM1 and another layer of the hot melt adhesive HM2 are provided on the underside surface of the absorber 56 across the entire region having the compressed portions 51. In this way, the above mentioned amount of the hot melt adhesives HM can be attained. Such structure is formed as follows. First, as illustrated in Fig. 13, the first hot melt adhesive HM1 is applied over a substantially entire surface facing the absorber 56 of the internal surface of the wrapping nonwoven fabric 58 in the spread state. After that, the absorber 56 is disposed on the first hot melt adhesive HM1 applied on the wrapping nonwoven fabric 58 at an intermediate portion thereof in the width direction WD, such that the top surface of the absorber 56 and the wrapping nonwoven fabric 58 are bonded to each other by the first hot melt adhesive HM1. Then, the second hot melt adhesive HM2 is applied over the substantially entire underside surface of the absorber 56. After that, as illustrated in Fig. 14, protruded portions of the wrapping nonwoven fabric 58 from both side edges of the absorber 56 are folded back to the underside surface of the absorber 56, as folded portions of the wrapping nonwoven fabric 58. In this way, these folded portions of the wrapping nonwoven fabric 58 and the underside surface of the absorber 56 are bonded to each other by the first hot melt adhesive HM1 and the second hot melt adhesive HM2. In addition, the overlapped portions of the wrapping nonwoven fabric 58 are bonded to each other by the first hot melt adhesive HM1. Finally, as illustrated in Fig. 9, the lattice shaped compressed portions 51 are formed by embossing.

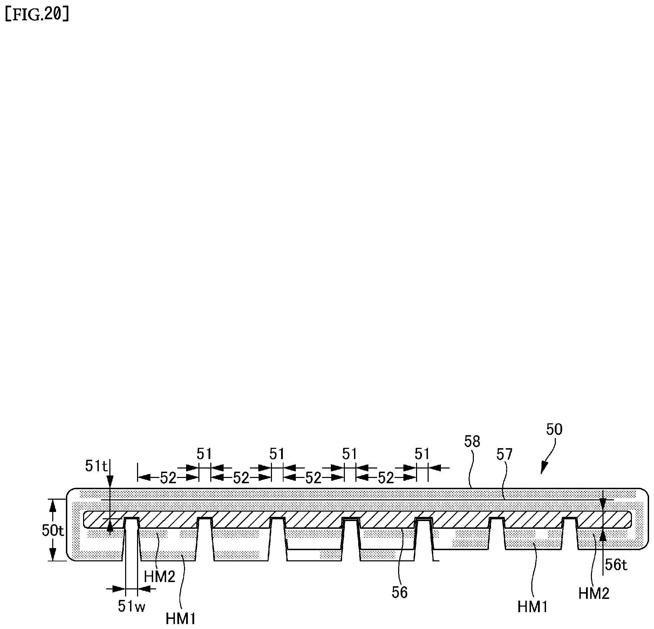

In order to reduce costs, as illustrated in Fig. 9 and the like, the absorbent element 50 may not have any another sheet layer such as a paper sheet layer, a nonwoven fabric sheet layer, etc. between the wrapping nonwoven fabric 58 and the absorber 56 (it is needless to say that an adhesive layer may be provided therebetween). Alternatively, the absorbent element 50 may have another sheet layer on at least one of the top side and underside of the absorber 56. For example, it is known that the compressed portion 51 of the absorbent element 50 has a higher absorbing ability and higher liquid holding capacity than the non-compressed portion 52. Accordingly, in order to prevent returning and in order not to feel wetness in a wearing state, the compressed portion 51 is preferably far from a wearer's body surface. However, in the present example, since the compressed portion 51 is a portion compressed in the thickness direction so as to be depressed from the underside surface of the absorbent element 50 into the absorber 56, the compressed portion 51 is closer to the wearer's body surface compared to a compressed portion 51 which would be formed oppositely in a top side-underside direction. In particular, as explained later, in a case where a part of the absorbent element 50 is uncovered such that the wrapping nonwoven fabric 58 is exposed on the top surface of the diaper at the uncovered part of the absorbent element 50, the compressed portion 51 is further closer to the wearer's body surface. Thus, as illustrated in Fig. 20, a returning-prevention layer 57 may be provided between the top surface (body surface side surface) of the absorber 56 and the underside surface (non-body surface side surface) of the wrapping nonwoven fabric 58. For the returning-prevention layer 57, a nonwoven fabric can be used appropriately which is hydrophobic or hydrophilic and has a density lower than the absorber 56. The thickness of the returning-prevention layer 57 can be determined as appropriate, but in a normal case, the thickness may be set to about 0.1 to 1.2 mm. The returning-prevention layer 57 in the illustrated example is disposed at the center so as to be shorter than the width of the absorber 56, but may be disposed over the maximum width of the absorber 56. Where the returning-prevention layer 57 is disposed in the front-back direction LD can be also determined as appropriate, and for example, it can be disposed over the maximum length of the absorber 56 or the maximum length of the absorbent element 50, alternatively, it can be disposed only at an intermediate portion in the front-back direction LD including the crotch part M.

### (Rising gather)

The rising gather 60 has a rising portion 68 rising from a side of the inner member 200, and the rising portion 68 comes into contact with a range of the wearer's body from an inguinal portion to a gluteal region through a periphery of the leg in order to prevent side leakage. In the rising gather 60 in the illustrated example, a root side portion 60B rises obliquely toward the center in the width direction WD, and a tip side portion 60A, which is closer to a tip portion of the rising gather 60 than an intermediate portion thereof, rises obliquely toward the outside in the width direction. However, a structure of the rising gather 60 is not limited to this example and it can be changed as appropriate, for example, the rising gather 60 rises, as a whole, toward the center in the width direction.

More specifically, the rising gather 60 in the illustrated example is formed as follows. That is, at a portion to be a tip of the rising gather 60, a belt-shaped gather sheet 62, which has a length equal to the length of the inner member 200 in the front-back direction, is folded back in the width direction WD to be folded in two, and then, a plurality of elongated gather elastic members 63, which are arranged along a longitudinal direction in a stretched state at intervals in the width direction WD, is fixed between two sheets of the sheet-folded portion and the vicinity thereof. A base portion of the rising gather 60 opposite to the tip portion thereof (an end portion opposite to the sheet-folded portion in the width direction WD) refers to a root portion 65 fixed to the side of the inner member 200, and a portion other than the root portion 65 refers to a main unit portion 66 (a portion on a folded portion side) extending from the root portion 65. In addition, the main unit portion 66 has the root side portion 60B, which extends toward the center in the width direction, and the tip side portion 60A, which is folded back at a tip of the root side portion 60B so as to extend toward the outside in the width direction. Then, a front end portion and a back end portion of the main unit portion 66 are referred to as fallen portions 67 fixed to a top surface of the side portion of the top sheet 30 in a state of falling down. Meanwhile, an intermediate portion located between the fallen portions 67 in the front-back direction refers to the non-fixed rising portion 68, and the gather elastic members 63 extending along the front-back direction LD are fixed in a stretched state to at least a tip portion of the rising portion 68.

In the rising gathers 60 configured as described above, as indicated by arrows in Fig. 3, the rising portions 68 rise so as to come into contact with the wearer's body surface by contraction forces of the gather elastic members 63. In particular, when the root portions 65 are located on an underside of the inner member 200, since the rising portions 68 rise so as to open toward the outside in the width direction in the crotch part and the vicinity thereof, the rising gathers 60 are brought into surface contact with the peripheries of the wearer's legs and the fitting property of the crotch part is thereby improved. The root portions 65 may be fixed to a top side of the inner member 200, e.g., a top surface of both the side portions of the top sheet 30.

As in the rising gather 60 in the illustrated example, in a bending structure where the main unit portion 66 includes the root side portion 60B, which extends toward the center in the width direction, and the tip side portion 60A, which is folded back at the tip of the root side portion 60B so as to extend toward the outside in the width direction, in each of the fallen portions 67, the tip side portion 60A and the root side portion 60B are bonded to each other in the state of falling down, and the root side portion 60B is bonded to the top sheet 30 in the state of falling down. In the fallen portion 67, for bonding facing surfaces to each other, at least one of a hot melt adhesive by various application methods and a means by material welding such as heat sealing or ultrasonic sealing can be used. In this case, bonding the root side portion 60B and the top sheet 30 to each other and bonding the tip side portion 60A and the root side portion 60B to each other may be performed by the same means or by different means. For example, it is preferable that the root side portion 60B and the top sheet 30 are bonded to each other by the hot melt adhesive and the tip side portion 60A and the root side portion 60B are bonded to each other by material welding.

As the gather sheet 62, a soft nonwoven fabric having excellent uniformity and concealability, such as a spunbonded nonwoven fabric (SS, SSS, or the like), an SMS nonwoven fabric (SMS, SSMMS, or the like), or a meltblown nonwoven fabric, which may be subjected to a water repellent treatment with silicone or the like as necessary can be used suitably. In this case, the nonwoven fabric preferably has a fiber basis weight of about 10 to 30 g/m². Alternatively, it is possible to use the gather sheet 62 folded in two so as to form two sheets, between which waterproof film is disposed, although not illustrated.

As the gather elastic member 63, a rubber thread or the like can be used. When a spandex rubber thread is used, the spandex rubber thread preferably has a fineness of 470 to 1240 dtex, and more preferably 620 to 940 dtex. The rubber thread preferably has a stretch rate of 150 to 350%, preferably 200 to 300% in a state where the gather elastic member 63 is fixed. The number of the gather elastic members 63 is preferably 2 to 6, and more preferably 3 to 5. A disposition interval of the gather elastic members 63 is suitably 3 to 10 mm. With such a configuration, a range where the gather elastic members 63 are disposed easily comes into surface contact with the wearer's body surface. The gather elastic members 63 may be disposed not only on a tip side but also on a root side.

In the rising portion 68 of the rising gather 60, at least one of a hot melt adhesive by various application methods and a means by material welding such as heat sealing, ultrasonic sealing, etc. can be used for adhering an internal layer of the gather sheet 62 and an external layer of the gather sheet 62 to each other or fixing the gather elastic members 63 interposed therebetween. It is preferred that the portions other than bonded portions of the gather elastic members 63 not be bonded or be weakly bonded, because adhering of the entire faces each other of the inner layer and the outer layer of the gather sheet 62 impairs flexibility. The illustrated example has the following structure. That is, the hot melt adhesive is applied only to outer peripheral surfaces of the gather elastic members 63 using application means such as comb gun or Surewrap nozzle, etc. and the gather elastic members 63 are interposed between the inner layer and the outer layer of the gather sheet 62, and by doing so, with only the hot melt adhesive applied to the outer peripheral surfaces of the gather elastic members 63, fixing of the gather elastic members 63 to the inner layer and the outer layer of the gather sheet 62 as well as fixing the inner layer and the outer layer of the gather sheet 62 can be performed.

Similarly, for fixing the fallen portion 67, at least one of a hot melt adhesive by various application methods and a means by material welding such as heat sealing or ultrasonic sealing can be used.

### (Side flap)

As illustrated in Fig. 1 to Fig. 4 and the like, on both the sides of the inner member 200, side flaps 70 protrude laterally from the absorber 56 and side stretchable regions SG stretching and contracting in the front-back direction are preferably formed in the side flaps 70 respectively. The side flap 70 in the illustrated example includes one or a plurality of mutually spaced elongated side elastic members 73 which are arranged along the front-back direction LD, a first sheet layer 71 which faces an external side of the side elastic member 73, and a second sheet layer 72 which faces an internal side of the side elastic member 73.

Sheet materials forming the first sheet layer 71 and the second sheet layer 72 are not particularly limited, and it is possible, as the sheet materials, to select appropriate nonwoven fabrics such as those available for the above mentioned rising gather 60 and the above mentioned outer member 12F, 12B. In the illustrated example, the gather sheet 62 of the rising gather 60 extends laterally so as to form the first sheet layer 71 and the second sheet layer 72, as discussed later. In this case, the front and back ends of the side flap 70 correspond to the front and back ends of the rising gather 60 (that is, in this case, front and back ends of the inner member 200).

As the side elastic member 73, an elongated elastic member similar to the above mentioned gather elastic member 63 may be used, without any particular limitation. The side elastic member 73 preferably has a stretch rate of 150 to 350%, and more preferably 200 to 270% in a state where the side elastic member 73 is fixed. The number of the side elastic members 73 is preferably 2 to 16, and more preferably 6 to 10. A disposition interval of the side elastic members 73 is suitably 5 to 10 mm.

The side elastic member 73 is fixed to the first sheet layer 71 and the second sheet layer 72. A hot melt adhesive HM by various application methods and a means by material welding such as heat sealing, ultrasonic sealing, etc. can be used for adhering of the first sheet layer 71 and the second sheet layer 72 to each other or fixing of the side elastic member 73 interposed therebetween. It is preferred that the portions other than a bonded portion of the side elastic member 73 not be bonded or be weakly bonded, because a large area of a portion where the first sheet layer 71 and the second sheet layer 72 are bonded to each other impairs flexibility. The illustrated example has the following structure. That is, the hot melt adhesive HM is applied only to an outer peripheral surface of the side elastic member 73 using application means such as comb gun or Surewrap nozzle, etc. and the side elastic member 73 is interposed between the first sheet layer 71 and the second sheet layer 72, and by doing so, with only the hot melt adhesive HM applied to the outer peripheral surface of the side elastic member 73, fixing of the side elastic member 73 to the first sheet layer 71 and the second sheet layer 72 as well as fixing the first sheet layer 71 and the second sheet layer 72 can be performed.

Further, in the illustrated example, in the side flap 70, a sheet material forming the first sheet layer 71 and a sheet material forming the second sheet layer 72 are folded back at a side edge of the side flap 70 to form a folded portion, which is fixed to an underside surface of the liquid impervious sheet 11 (in an enveloping structure). As in the illustrated example, such fixing can be performed by the hot melt adhesive HM. Alternatively, it can be performed also by a material welding.

The side flaps 70 can be omitted.

### (Outer Member)

The outer member 12F, 12B is composed of the front outer member 12F, which is a rectangular shaped member forming at least the lower torso portion in the front body F, and the back outer member 12B, which is a rectangular shaped member forming at least the lower torso portion in the back body B respectively. The front outer member 12F and the back outer member 12B may not continuous on a crotch side but may be separated in the front-back direction LD (outer member separated type), or the front outer member 12F and the back outer member 12B may extend continuously from the front body to the back body (outer member integrated type), although not illustrated. In an outer member separated type underpants type disposable diaper, the separation distance 12d in the front-back direction LD can be set to, for example, about 40 to 60% of the maximum length Y of the diaper. In the illustrated example, a lower edge of the front outer member 12F and a lower edge of the back outer member 12B may be linear shaped along the width direction WD. However, at least one of the lower edge of the front outer member 12F and the lower edge of the back outer member 12B may be curved so as to follow peripheries of the wearer's legs.

In the outer member separated type underpants type disposable diaper, since the inner member 200 is exposed between the front outer member 12F and the back outer member 12B, in order to prevent the liquid impervious sheet 11 from being exposed on the underside surface of the inner member 200, the cover nonwoven fabric 13 is favorably provided that covers the underside surface of the inner member 200 from an overlapping portion between the front outer member 12F and the inner member 200 to an overlapping portion between the back outer member 12B and the inner member 200. An internal surface of the cover nonwoven fabric 13 and an external surface of the cover nonwoven fabric 13 can be bonded to respective facing surfaces by the hot melt adhesive. A nonwoven fabric used for the cover nonwoven fabric 13 can be selected as appropriate, for example, a nonwoven fabric similar to the nonwoven fabric forming the outer member 12F, 12B. Note that the outer member may be continuous from the front body F to the back body B through the crotch part M, although not illustrated. In this case, the outer member may have not only a part corresponding to the lower torso region T but also a part corresponding to the intermediate region L.

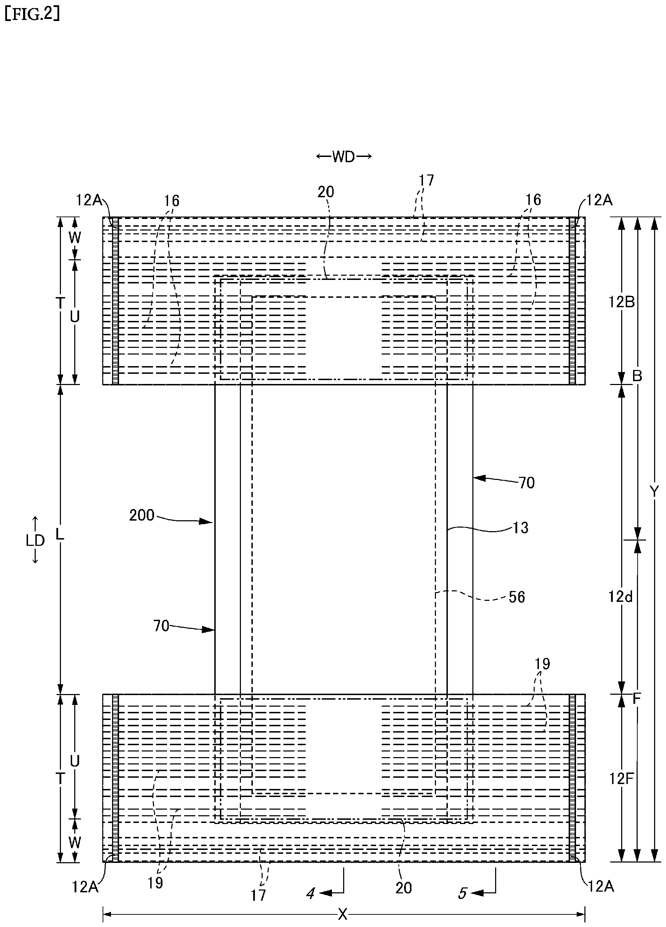

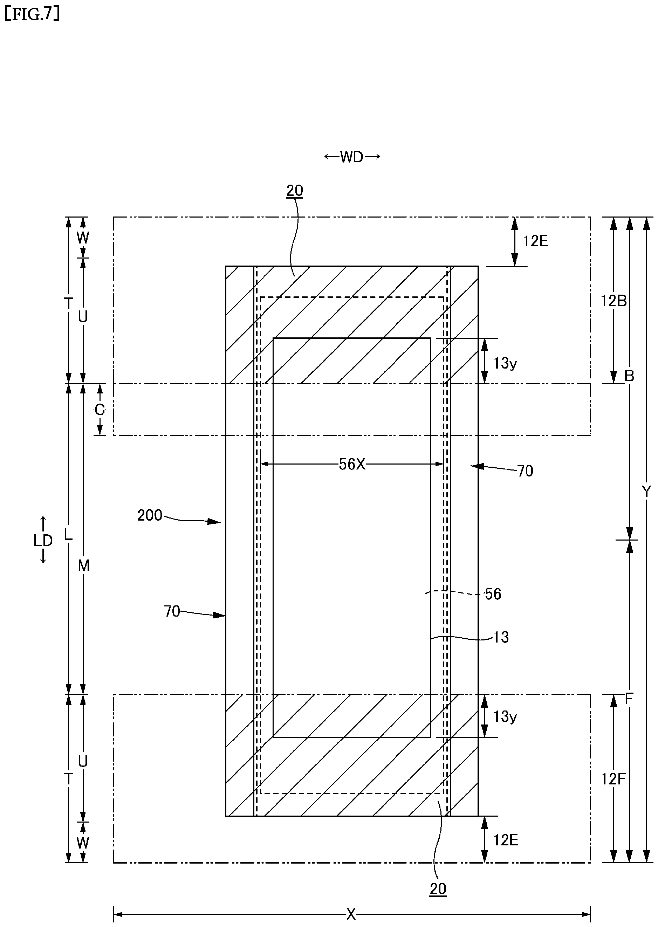

The front outer member 12F and the back outer member 12B have, on the front side and the back side, the lower torso portion forming the lower torso region T. In the example illustrated in Fig. 1 and Fig. 2, a dimension of the front outer member 12F is the same as a dimension of the back outer member 12B in the front-back direction L, and the front outer member 12F and the back outer member 12B do not have a part corresponding to the intermediate region L. Meanwhile, as illustrated in Fig. 7, it is possible that a dimension of the back outer member 12B is longer than a dimension of the front outer member 12F in the front-back direction LD, and the front outer member 12F does not have a part corresponding to the intermediate region L, but the back outer member 12B has a gluteal cover portion C, which protrudes from the lower torso region T so as to extend in the intermediate region L. The front outer member 12F may have also an inguinal cover portion protruding from the lower torso region T so as to extend in the intermediate region L, although not illustrated.

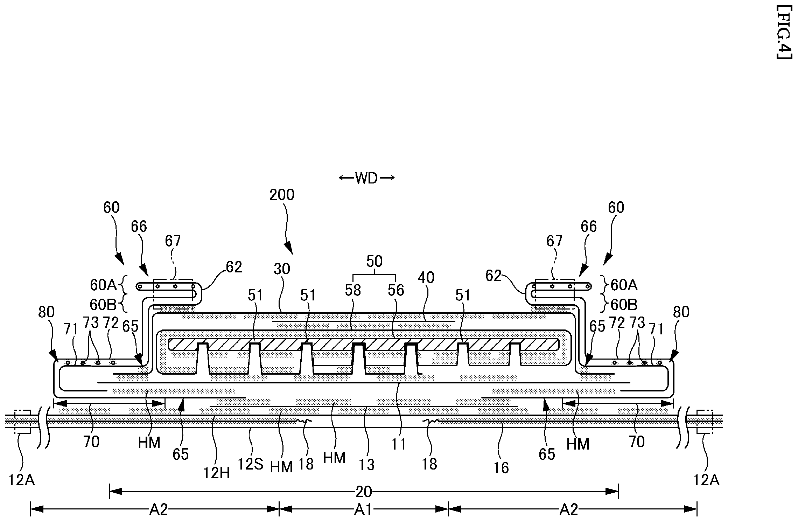

As illustrated in Fig. 4 and Fig. 5, the outer member 12F, 12B is formed by bonding an outer sheet layer and an inner sheet layer, which are adjacent to elastic members 16 to 19 described later on an outer side and an inner side thereof respectively, by the bonding means such as a hot melt adhesive or welding. The outer sheet layer and the inner sheet layer may be composed of two individual sheet materials 12S, 12H, as in the illustrated example. Alternatively, the outer sheet layer and the inner sheet layer may be composed of a common single sheet material. In this latter case, for example, the inner sheet layer and the outer sheet layer are formed by an inner portion and an outer portion of a single sheet of a sheet material folded back at an edge of the waist opening WO (which may be an edge of the lower torso region on the crotch side) in a part or whole of the outer member 12F, 12B respectively. The illustrated example corresponds to an example in that former case, and the sheet material 12S forming the outer sheet layer in the under-waist end portion goes around the sheet material 12H forming the inner sheet layer in the under-waist end portion on the waist opening WO side and folded back inward. An folded-back portion 12r extends so as to cover an end portion of the inner member 200 on the waist opening WO side. Meanwhile, in the waist end portion, the folded-back portion 12r corresponds to an inner sheet layer adjacent to the elastic members on the inner side thereof.

In order to enhance a fitting property of the outer member 12F, 12B to a wearer's lower torso, the elastic members 16 to 19 are incorporated therein so as to form a stretchable region A2, which elastically stretches and contracts in the width direction WD due to stretching and contracting of the elastic members 16 to 19. In the stretchable region A2, in a natural length state, the outer member 12F, 12B contracts due to contraction of the elastic members to form wrinkles or pleats. When the outer member 12F, 12B stretches in a longitudinal direction of the elastic members, the outer member 12F, 12B can be stretched to a predetermined stretch rate at which the outer member 12F, 12B is spread without wrinkles. As each of the elastic members 16 to 19, in addition to an elongated elastic member (illustrated example) such as a rubber thread, a known elastic member such as a belt-shaped member, a net-shaped member, or a film-shaped member can be used without particular limitation. Each of the elastic members 16 to 19 may be formed of either a synthetic rubber or a natural rubber may be used.

The elastic members 16 to 19 in the illustrated example will be described in more detail. In the waist end portion W of the outer member 12F, 12B, a plurality of waist end portion elastic members 17 is arranged at intervals in the front-back direction so as to be continuous over the entire width direction WD. Among the waist end portion elastic members 17, one or more waist end portion elastic members 17 disposed in a region adjacent to the under-waist end portion U may overlap with the inner member 200, or may be disposed on both sides thereof in the width direction except for the center in the width direction overlapping with the inner member 200. As the waist end portion elastic members 17, it is preferable to dispose about 2 to 15, particularly about 4 to 10 rubber threads each having a fineness of about 155 to 1880 dtex, particularly about 470 to 1240 dtex (in a case of a synthetic rubber, and having a cross section of about 0.05 to 1.5 mm², particularly about 0.1 to 1.0 mm² in a case of a natural rubber) at intervals of 2 to 12 mm, particularly 3 to 7 mm. A resultant stretch rate of the waist end portion W in the width direction WD is preferably about 150 to 400%, and particularly preferably about 220 to 320%. In the waist end portion W, all of the elastic members 17 in the front-back direction LD do not have to have the same fineness and do not have to have the same stretch rate. For example, the fineness of the elastic members 17 partly may be different each other and the stretch rate thereof partly may be different each other.

In addition, in the under-waist end portion U of the outer member 12F, 12B, a plurality of elongated under-waist end portion elastic members 16 and 19 is arranged at intervals in the front-back direction so as to form an under-waist end stretchable region (a region having the under-waist end portion elastic members 16 and 19). As the under-waist end portion elastic members 16 and 19, it is preferable to dispose about 5 to 30 rubber threads each having a fineness of about 155 to 1880 dtex, particularly about 470 to 1240 dtex (in a case of a synthetic rubber, and having a cross section of about 0.05 to 1.5 mm², particularly about 0.1 to 1.0 mm² in a case of a natural rubber) at intervals of 1 to 15 mm, particularly 3 to 8 mm. A resultant stretch rate of the under-waist end portion U in the width direction WD is preferably about 200 to 350%, and particularly preferably about 240 to 300%. In the under-waist end portion U, all of the elastic members in the front-back direction LD do not have to have the same fineness and do not have to have the same stretch rate and the fineness of the elastic members partly may be different each other and the stretch rate thereof partly may be different each other.

In a case where the elastic members 16 and 19 are disposed in a range in the front-back direction having the absorber 56 as in the under-waist end portion U in the illustrated embodiment, in order to prevent contraction of the absorber 56 in the width direction WD in a part or the whole thereof, it is preferable that, as illustrated in Fig. 4, Fig. 5, Fig. 12 and the like, a middle portion in the width direction of the under-waist end portion U including a part or whole of a portion overlapping with the absorber 56 in the width direction WD, refer to a non-stretchable region A1, and both side portions of the non-stretchable region A1 in the width direction refer to the stretchable region A2 (an under-waist end portion stretchable region in the illustrated example). Dimensions in the width direction of the stretchable region A2 disposed at both the sides of the front body part and the back body part of the non-stretchable region A1 in the width direction can be substantially constant in the front-back direction LD respectively as in the illustrated example. Alternatively, these dimensions can be changed in the front-back direction LD, although not illustrated. In addition, the dimensions in the width direction WD of the stretchable region A2 disposed at both the sides of the front body part of the non-stretchable region A1 in the width direction WD can be substantially the same as or different from the dimensions in the width direction WD of the stretchable region A2 disposed at both the sides of the back body part of the non-stretchable region A1 in the width direction WD.

Such stretchable region A2 and the non-stretchable region A1 can be formed as follows. That is, elastic members 16 to 17 and 19 are fixed between the inner sheet layer and the outer sheet layer, and after that, the elastic members 16, 19 are cut at one place or over the whole in an intermediate portion in the width direction in a region to be the non-stretchable region A1, by means of finely cutting or by means of pressing and heating of the elastic members 16, 19 so as to destroy elasticity in the non-stretchable region A1, while in a region to be the stretchable region A2, elastic members 16, 19 are left as they are so as not to destroy elasticity in the stretchable region A2. Meanwhile, it turns out that idle elastic members 18, which do not substantially contribute to generate elasticity, are remained in the stretchable region A2.

As the sheet material 12H forming the inner sheet layer and the sheet material 12S forming the outer sheet layer, any materials can be used without particular limitation. However, a nonwoven fabric is preferably used. In a case where the nonwoven fabric is used, a basis weight per one sheet thereof is preferably about 10 to 30 g/m².

The elastic members 16 to 19 can be fixed to the outer member 12F, 12B by a known method. In addition, the inner sheet layer and the outer sheet layer can be also bonded to each other by a known method. For example, in portions having the elastic members 16 to 19 in the outer member 12F, 12B, the hot melt adhesive HM is applied only to an outer peripheral surface of each of the elastic members 16 to 19 using application means such as comb gun or Surewrap nozzle, etc. and the elastic members 16 to 19 are interposed between the inner sheet layer and the outer sheet layer, and by doing so, with only the hot melt adhesive HM applied to the outer peripheral surfaces of the elastic members 16 to 19, fixing of the elastic members 16 to 19 to the inner sheet layer and the outer sheet layer as well as fixing the inner sheet layer and the outer sheet layer can be performed.

### (Inner member bonded portion)

The inner member 200 can be fixed to the outer member 12F, 12B by the bonding means by material welding such as heat sealing or ultrasonic sealing, or by a hot melt adhesive. In the illustrated example, via the hot melt adhesive applied to the underside surface of the inner member 200, that is, in this case, to the underside surface of the liquid impervious sheet 11 and root portions 65 of the rising gather 60, the inner member 200 is fixed to the inner surface of the outer member 12F, 12B. An inner member bonded portion 20 for fixing the inner member 200 to the outer member 12F, 12B can be disposed in almost the entire region where the inner member 200 overlaps with the outer member 12F, 12B as illustrated in Fig. 2. Alternatively, the inner member bonded portion 20 can be disposed, for example, in a portion excluding both end portions of the inner member 200 in the width direction.

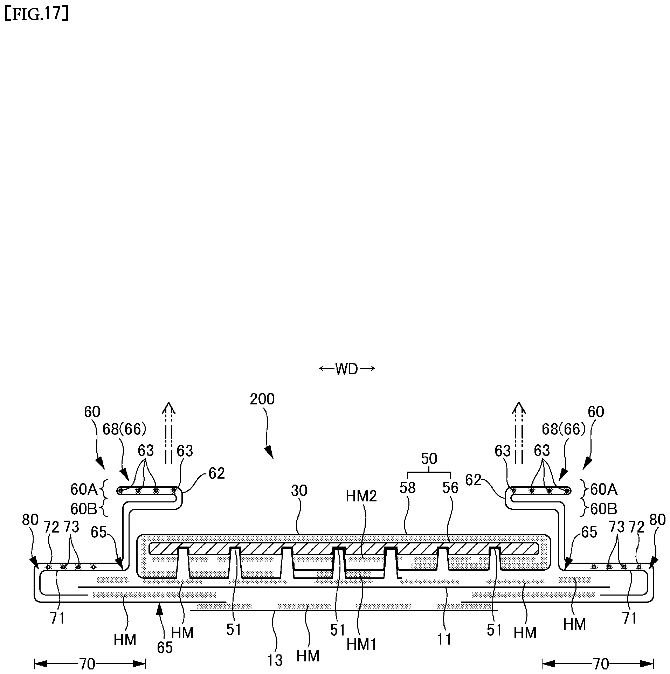

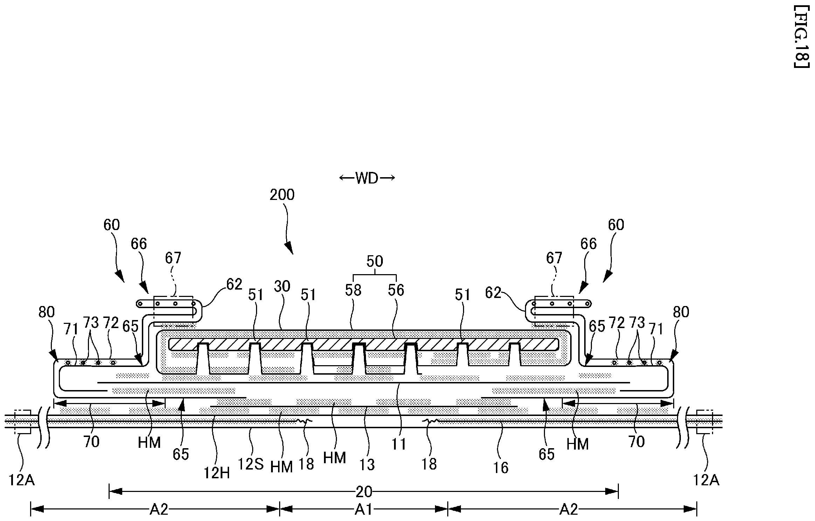

As stated above, since the escape of the super absorbent polymer particles through the wrapping nonwoven fabric 58 can be suppressed, it is possible to omit a member such as the top sheet 30 for covering the top surface of the absorbent element 50 and the intermediate sheet 40 as illustrated in Fig. 17 and Fig. 18, thus a significant cost reduction can be attained. In this case, as long as an entire range in the front-back direction of a middle part of the top surface of the absorbent element 50 excluding both end portions thereof in the width direction WD is uncovered by any other member, such that the wrapping nonwoven fabric 58 is exposed on the top surface of the absorbent article at the uncovered part of the absorbent element 50, only the middle part of the top surface of the absorbent element 50 in the width direction WD may be uncovered by any other member as in the illustrated example, alternatively, the entire top surface of the absorbent element 50 may be uncovered by any other member.

### <Effect evaluation test>

Sample 1 to Sample 8 of underpants-type disposable diapers are prepared each of which has a similar structure as that of the diaper of Fig. 1 to Fig. 6 except that the top sheet 30 and the intermediate sheet 40 are omitted and a width of an bonding region for bonding the absorbent element 50 and the liquid impervious sheet 11 is increased as illustrated in Fig. 17 and Fig. 18. Among Sample 1 to Sample 8, other specifications are common except those different from each other in Table 1.

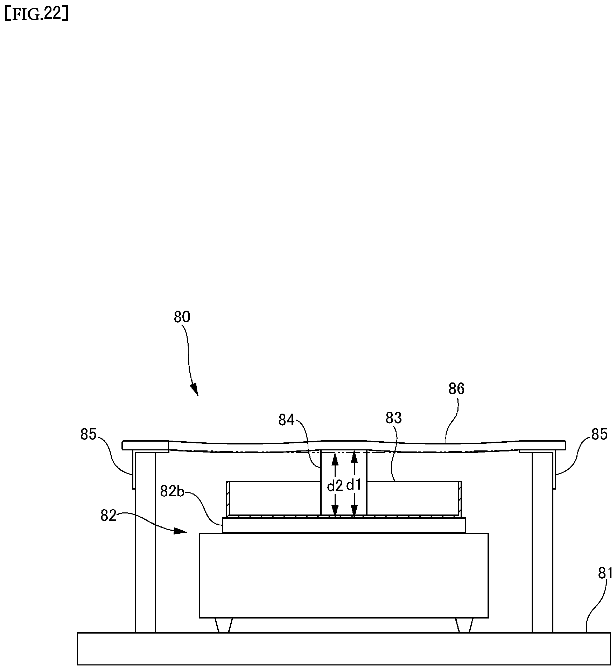

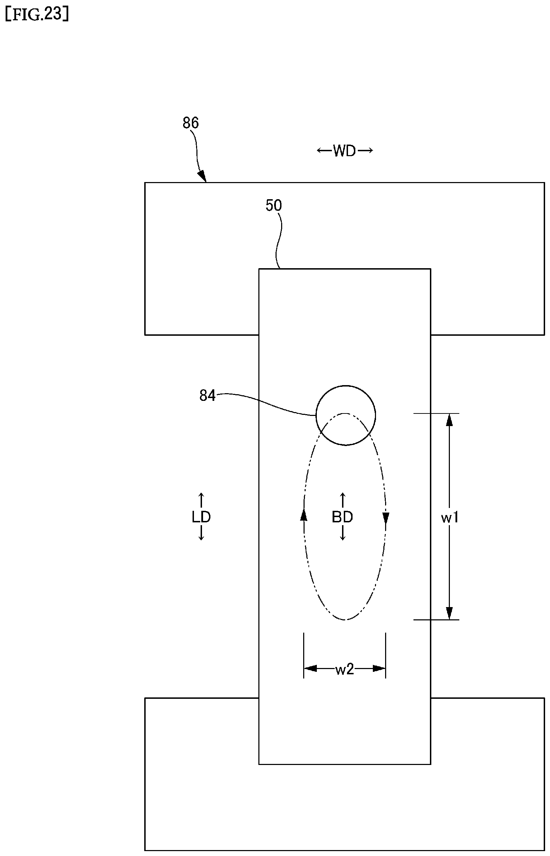

A test facility 80 is illustrated in Fig. 22. This facility 80 is configured as follows. That is, a collecting tray 83, which is provided for receiving super absorbent polymer particles, is fixed on a shaking stage 82b of a shaker 82 (MK161 of Yamato Scientific Co., Ltd.), which is installed on a horizontal plane 81, and a friction member 84 formed of a metal column (diameter of 55 mm) is secured upright on a center of the collecting tray 83 such that the collecting tray 83 and the friction member 84 are shaken together with the shaking stage 82b integrally. A distance d1 in a vertical direction between an upper surface of the friction member 84 and an upper surface of the shaking stage 82b is 72 mm. A pair of supporting pillars 85 are secured upright on the horizontal plane 81 on both lateral sides of the shaker 82, respectively. Each sample 86 in the spread state (as the state illustrated in Fig. 1) is laid between the supporting pillars 85 and secured thereto, while a top surface (on which a wrapping nonwoven fabric is exposed) of the sample 86 faces downward. In this case, the front-back direction LD of the sample 86 corresponds to a major axis direction BD of an elliptical shaking mode. Further, a distance d2 in the vertical direction between a portion of the top surface of the sample 86 which is to be brought into contact with the friction member 84 and the upper surface of the shaking stage 82b would be 71 mm in a measurement condition without the friction member 84, which makes it possible for the top surface of the sample 86 to be brought into contact with the friction member 84. Then, the shaking mode of the shaker 82 is set to be elliptical (shaking range w1 of major axis: 30 mm, shaking range w2 of minor axis: 20 mm), the shaker 82 is shaken for 2 minutes at the shaking frequency of 90 rpm, and the friction member 84 is moved in the elliptical shaking mode as illustrated in Fig. 23 such that the friction member 84 is rubbed against the top surface of the sample 86. After that, number of the super absorbent polymer particles, which are fallen on the collecting tray 83, is counted.

The result of the test is indicated in Table 1. In the wrapping nonwoven fabrics of Sample 1, Sample 3 and Sample 5, the super absorbent polymer particles are escaped. However in the wrapping nonwoven fabrics of Sample 2, Sample 4 and Sample 6, any of the super absorbent polymer particles are not escaped, in spite of their high permeabilities. Also in the wrapping nonwoven fabrics of Sample 7 and Sample 8, any of the super absorbent polymer particles are not escaped, due to their high basis weights and low air permeabilities.

| Sample No. | | 1 | 2 | 3 | 4 | 5 | 6 | 7 | 8 |
|---|---|---|---|---|---|---|---|---|---|
| Wrapping nonwoven fabric | Type | SMS nonwoven fabric | SMS nonwoven fabric | SMS nonwoven fabric | SMS nonwoven fabric | SMS nonwoven fabric | SMS nonwoven fabric | SMS nonwoven fabric | SMS nonwoven fabric |
| | Fiber bonding method | Point thermal bonding | Point thermal bonding | Point thermal bonding | Point thermal bonding | Point thermal bonding | Point thermal bonding | Point thermal bonding | Point thermal bonding |
| | Air permeability, measured according to Method A(cm³/(cm²·sec)) | 66.9 | ← | 45.2 | ← | 25.9 | ← | 25.0 | ← |
| | Basis weight (g/m²) | 10 | ← | 13 | ← | 15 | ← | 17 | ← |
| Absorber | Basis weight of pulp (g/m²) | 158 | ← | ← | ← | ← | ← | ← | ← |
| | Basis weight of SAP (g/m²) | 163 | ← | ← | ← | ← | ← | ← | ← |
| | Pulp : SAP | 49 : 51 | ← | ← | ← | ← | ← | ← | ← |
| | thickness (mm) | 4.29 | 4.29 | 4.29 | 4.29 | 4.30 | 4.30 | 4.31 | 4.31 |
| Compressed portion | Pattern | Figs. 10 and 11 | ← | ← | ← | ← | ← | ← | ← |
| | Depressed portions are formed on the top surface or the underside surface of the absorbent element | top surface | underside surface | top surface | underside surface | top surface | underside surface | top surface | underside surface |
| | Area ratio (%) | 13 | ← | ← | ← | ← | ← | ← | ← |
| Number of escaped SAP | | 6 | 0 | 4 | 0 | 1 | 0 | 0 | 0 |

| | | | | | | | | | |
|---|---|---|---|---|---|---|---|---|---|
| SAP•••Super absorbent polymer particles | | | | | | | | | |

### <Description of Terms Used in Specification>

The following terms used in the specification have the following meanings unless otherwise specified in the specification.

- "Front-back direction" refers to a direction (longitudinal direction) indicated by a reference sign LD in the drawings, "width direction" means a direction (left-right direction) indicated by a reference sign WD in the drawings, and the front-back direction and the width direction are orthogonal to each other.
- "Top side" refers to a side closer to a wearer's body surface when an absorbent article is worn, and "underside" means a side far from a wearer's body surface when the absorbent article is worn.
- "Top surface" refers to a surface of a portion closer to a wearer's skin when an absorbent article is worn, and "underside surface" refers to a surface of a portion far from the wearer's skin when the absorbent article is worn.
- "Stretch rate" refers to a value when the natural length is 100%. For example, a stretch rate of 200% is synonymous with an elongation ratio of 2.
- "Artificial urine" is a mixture of urea: 2 wt%, sodium chloride: 0.8 wt%, calcium chloride dihydrate: 0.03 wt%, magnesium sulfate heptahydrate: 0.08 wt%, and ion exchanged water: 97.09 wt%.
- "Gel strength" is measured as follows. 1.0 g of super absorbent polymers is added to 49.0 g of artificial urine and the mixture is agitated with a stirrer. The resulting gel is left in a thermo-hygrostat at 40°C x 60%RH for three hours and then cooled to room temperature. The gel strength of the gel is measured with a curdmeter (Curdmeter-MAX ME-500 manufactured by I. Techno Engineering Co., Ltd).
- "Average fiber diameter" is obtained as follows. A target layer is observed under a microscope (optical microscope, SEM, or the like), with 1000 magnification such that fiber diameters (um) of randomly selected 30 constituting fibers of the target layer are measured. Then, on the basis of these fiber diameters, an average is calculated as the average fiber diameter.
- "Basis weight" is measured as follows. A sample or a test piece is pre-dried, and then is left in a test room or a device in a standard state (temperature 23 ± 1°C, relative humidity 50 ± 2% in a test location), and is put in a constant weight state. Pre-drying refers to setting the weight of the sample or the test piece to a constant weight in an environment in which temperature is 100°C. Incidentally, pre-drying is unnecessary for a fiber having an official moisture regain of 0.0%. A sample having dimensions of 100 mm × 100 mm is cut off from the test piece in the constant weight state using a sampling template (100 mm × 100 mm). A weight of the sample is measured and multiplied by 100 to calculate a weight per square meter, and the weight is set to the basis weight.
- The "thickness" of a thick member such as the absorber 56, the absorbent element 50, or the compressed portion 51 is measured using a thickness measuring instrument of Ozaki Mfg. Co., Ltd. (Peacock, Dial Thickness Gauge, Model J-B (measurement range of 0 to 35 mm, circular contact point with a diameter of 20 mm, measuring force of about 3.0 N, and pressure of about 30 KPa) by horizontally placing the sample and the thickness measuring device.
- The "thickness" of a thin member such as a nonwoven fabric is automatically measured under the condition of load: 0.098 N/cm² and pressure area: 2 cm² using an automatic thickness meter (KES-G5 handy compression measurement program). Incidentally, the "thickness" of the wrapping nonwoven fabric refers to the thickness thereof in a portion of the absorbent element 50 whose thickness 50t reaches the maximum value.
- The "thickness of a layer" of a nonwoven fabric is measured as follows. A cross section of a target layer in the portion of the absorbent element 50 whose thickness 50t reaches the maximum value is observed under a microscope (optical microscope, SEM, or the like), with 1000 magnification such that apparent thicknesses of randomly selected 5 measurement positions are measured. Then, on the basis of these thicknesses, an average is calculated as the thickness of the layer of the nonwoven fabric.
- The "area ratio" refers to a ratio of a target portion to a unit area, and is represented as a percentage by dividing a total area of target portions (for example, the compressed portions 51) in a target region (for example, the underside surface of the absorbent element 50) by an area of the target region.
- Water absorption capacity is measured in accordance with JIS K7223-1996 "Testing method for water absorption capacity of super absorbent polymers."
- Water absorption speed is defined as "time that elapse before the end point" measured with 2g of super absorbent polymers and 50g of normal saline solution in accordance with JIS K7224-1996 "Testing method for water absorption speed of super absorbent polymers."
- "Spread state" means a flatly spread state without contraction (including any contraction such as one due to an elastic member) or slack.
- The dimensions of the respective components are measured in the spread state, not the natural length state, unless otherwise specified.
- The tests and measurements are carried out in a test room or a test device under normal conditions (an ambient temperature of 23 ± 1°C and with a relative humidity of 50 ± 2% at the testing site), unless the environmental condition for the tests and measurements are otherwise specified.

### Industrial Applicability

The present invention can be applied for a general absorbent article including a disposable diaper such as an underpants type disposable diaper, a tape-type disposable diaper, or a pad type disposable diaper, in addition to a sanitary napkin or the like.

### Reference Signs List

- 11: Liquid impervious sheet
- 12A: Side seal portion
- 12B: Back outer member
- 12E: Waist end side extending portion
- 12F, 12B: Outer member
- 12F: Front outer member
- 12S, 12H: Sheet material
- 13: Cover nonwoven fabric
- 16, 19: Under-waist end portion elastic member
- 17: Waist end portion elastic member
- 18: Idle elastic member
- 20: Inner member bonded portion
- 200: Inner member
- 30: Top sheet
- 40: Intermediate sheet
- 50: Absorbent element
- 51: Compressed portion
- 51f: Unit frame
- 52: non-compressed portion
- 56: Absorber
- 56c: Covering layer
- 56L: Low-basis weight section
- 58: Wrapping nonwoven fabric
- 60: Rising gather
- 60A: Tip side portion
- 60B: Root side portion
- 62: Gather sheet
- 63: Gather elastic member
- 67: Fallen portion
- 68: Rising portion
- 70: Side flap
- 71: First sheet layer
- 72: Second sheet layer
- 73: Side elastic member
- A1: Non-stretchable region
- A2: Stretchable region
- B: Back body
- C: Gluteal cover portion
- F: Front body
- HM: Hot melt adhesive
- L: Intermediate region
- LD: Front-back direction
- LO: Leg opening
- M: Crotch part
- SG: Side stretchable region
- T: Lower torso region
- U: Under-waist end portion
- W: Waist end portion
- WD: Width direction
- WO: Waist opening
- HM1: First hot melt adhesive
- HM2: Second hot melt adhesive
- 57: Returning-prevention layer

## Claims

1. An absorbent article comprising:
a crotch part;
a front side part which extends forward from the crotch part;
a back side part which extends backward from the crotch part; and
an absorbent element including an absorber and a wrapping nonwoven fabric wrapping the absorber, the absorbent element being provided in a range in a front-back direction including the crotch part,
wherein the absorber has a layer formed by accumulating a mixture of pulp fibers and super absorbent polymer particles,
the wrapping nonwoven fabric includes a meltblown layer formed of fibers having an average fiber diameter of 5 um or less,
the wrapping nonwoven fabrics has an air permeability of 25.5 to 70.0 cm³/(cm²·s), measured according to Method A (Frazier method) specified in JIS L 1096 : 2010 in a state in which five layers of the nonwoven fabrics are stacked,
the absorbent element is provided with compressed portions arranged at intervals and compressed in a thickness direction so as to be depressed from an underside surface of the absorbent element into the absorber; and
the absorbent element is free of compressed portions compressed in the thickness direction so as to be depressed from a top surface of the absorbent element into the absorber.

2. The absorbent article according to claim 1,
wherein the wrapping nonwoven fabric includes the one or plural meltblown layers and one or plural protective layers,
constituent fibers of the protective layer have an average fiber diameter of 10 to 25 um,
the wrapping nonwoven fabric has an elongation rate of 20 to 100% in the front-back direction, measured according to a normal time method specified in JIS L 1913 : 2010, and
the wrapping nonwoven fabric has an elongation rate of 20 to 110% in a width direction, measured according to the normal time method specified in JIS L 1913 : 2010.

3. The absorbent article according to claim 1 or 2,
wherein, in the absorber, the pulp fibers have a basis weight of 100 to 450 g/m²,
in the absorber, a weight ratio of pulp fibers : super absorbent polymer particles is 30 : 70 to 70 : 30,
a maximum thickness of the absorbent element is 4 to 35 mm, and
a thickness of each of the compressed portions is 15 to 35% of the maximum thickness of the absorbent element.

4. The absorbent article according to claim 3,
wherein a width of each of the compressed portions is 1 to 3 mm, and
the compressed portions are formed in an oblique lattice shape including first portions each of which diagonally inclines at a clockwise angle of 40 to 50° with respect to the front-back direction in a plan view, and second portions each of which diagonally inclines at a counterclockwise angle of 40 to 50° with respect to the front-back direction in the plan view.

5. The absorbent article according to claim 1 to 2,
wherein the absorber includes, at a top surface thereof, a covering layer formed by accumulating only pulp fibers.

6. The absorbent article according to claim 1 to 2,
wherein the wrapping nonwoven fabric has:
a top surface-portion located on a top side of the absorber;
a first underside-portion, which continues from the top surface-portion and goes around the absorber on a one side edge thereof to be folded back and extended inwardly on an underside thereof; and
a second underside-portion, which continues from the top surface-portion and goes around the absorber on an other side edge thereof to be folded back and extended inwardly on the underside thereof,
a tip side portion of the first underside-portion and a tip side portion of the second underside-portion are overlapped with each other so as to form a stacked portion, and
all of the compressed portions are formed within the stacked portion.

7. The absorbent article according to claim 1 or 2,
wherein an entire range in the front-back direction of a middle part of the absorbent element excluding both end portions thereof in the width direction is uncovered by any other member, such that the wrapping nonwoven fabric is exposed on a top surface of the absorbent article.

8. The absorbent article according to claim 1 or 2
wherein a liquid impervious sheet is attached to the underside surface of the absorbent element via a hot melt adhesive,
the liquid impervious sheet extends from a position on a front side of a front edge of the absorbent element to a position on a back side of a back edge of the absorbent element, and from a position on a left side of a left side edge of the absorbent element to a position on a right side of a right side edge of the absorbent element, and
the hot melt adhesive is applied from a position on a front side of a front edge of the absorber to a position on a back side of a back edge of the absorber, and from a position between a left side edge of the absorber and the left side edge of the absorbent element to a position between a right side edge of the absorber and the right side edge of the absorbent element.
